(19) Europäisches Patentamt
European Patent Office
Office européen des brevets

(11) Publication number: **0 060 607**
**B1**

(12) **EUROPEAN PATENT SPECIFICATION**

(45) Date of publication of patent specification: **28.08.85**

(51) Int. Cl.[4]: **C 07 D 241/44,**
C 07 D 241/52, A 01 N 43/60
// C07C43/23, C07C65/21,
C07C69/732, C07C69/734

(21) Application number: **82300074.0**

(22) Date of filing: **07.01.82**

(54) **2-(2-halo-4-(6-haloquinoxalin-2-yloxy)phenoxy)propionates, process for their synthesis, herbicidal compositions containing them and their use as herbicides.**

(30) Priority: **12.01.81 AU 7201/81**

(43) Date of publication of application:
**22.09.82 Bulletin 82/38**

(45) Publication of the grant of the patent:
**28.08.85 Bulletin 85/35**

(84) Designated Contracting States:
**AT BE CH DE FR GB IT LI LU NL SE**

(56) References cited:
**EP-A-0 023 785**
**DE-A-2 820 032**
**GB-A-2 042 539**

(73) Proprietor: **ICI AUSTRALIA LIMITED**
**1 Nicholson Street**
**Melbourne Victoria 3001 (AU)**

(72) Inventor: **Serban, Alexander**
**3 Maple Court**
**Doncaster Victoria, 3108 (AU)**
Inventor: **Watson, Keith Geoffrey**
**36 Medway Street**
**Box Hill North Victoria, 3129 (AU)**
Inventor: **Farquharson, Graeme John**
**10 Steane Street**
**Reservoir Victoria, 3073 (AU)**

(74) Representative: **Bishop, Nigel Douglas et al**
**Imperial Chemical Industries PLC Legal**
**Department: Patents PO Box 6**
**Welwyn Garden City Herts, AL7 1HD (GB)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description for the Contracting State: LU**

This invention relates to quinoxaline derivatives having biological activity, in particular having herbicidal properties, to processes for the preparation of such compounds, to intermediates useful in the preparation of such compounds and to herbicidal compositions and processes utilizing such compounds.

Accordingly the present invention provides a compound of formula I:

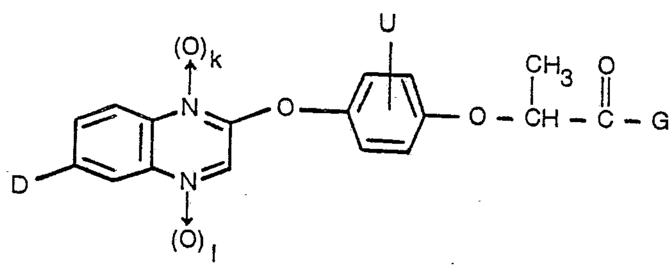

I

or a salt thereof wherein:

D and U are independently chosen from the group consisting of halogen, methyl and halomethyl;

G is chosen from the group consisting of hydroxy, mercapto, $C_1$ and $C_{10}$ alkoxy, $C_2$ to $C_{10}$ alkenyloxy, $C_3$ to $C_{10}$ alkynyloxy, $C_1$ to $C_{10}$ alkylthio, $C_2$ to $C_{10}$ alkenylthio, $C_3$ to $C_{10}$ alkynylthio, $C_3$ to $C_7$ cycloalkoxy and the group OM wherein M is an alkali metal or alkaline earth metal ion; and

k and l are independently chosen from 0 and 1.

In the compounds of formula I the 2-carbon atom of the propionate group is asymmetrically substituted and therefore the compound of formula I are optically active. The present invention includes the individual stereoisomers of such compounds, mixtures of those stereoisomers and the racemic mixture of stereoisomers. It is known in the art that certain stereoisomers are more herbicidally active than others, cf. for example, European Patent Published Application 0002800.

Preferred D include fluorine, chlorine, bromine, iodine and trifluoromethyl. More preferred D is fluorine, chlorine or bromine, especially fluorine or chlorine, particularly chlorine.

Preferred U include a fluorine, chlorine, bromine or iodine atom in the 2-position of the phenyl ring. More preferred U is 2-fluoro.

Preferred G include hydroxy, $C_1$ to $C_6$ alkoxy, $C_2$ to $C_6$ alkenyloxy, $C_3$ to $C_6$ alkynloxy, cyclohexyloxy and the group OM wherein M is an alkali metal ion. More preferred G include hydroxy and $C_1$ to $C_6$ alkoxy, especially hydroxy, ethoxy, *n*-propoxy and *n*-butoxy.

Examples of compounds embraced by the invention include:

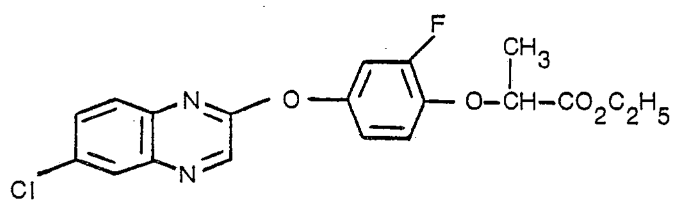

1

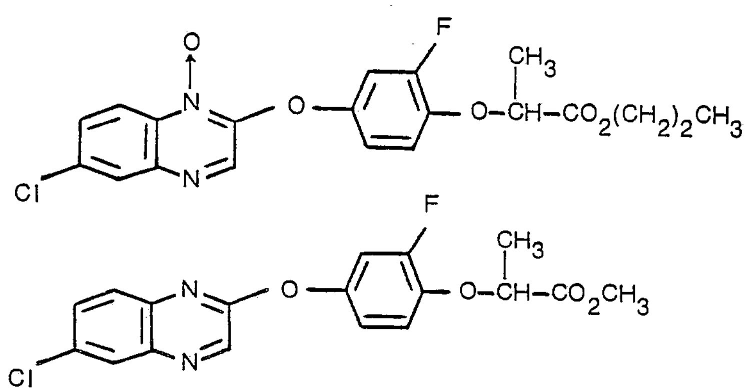

2

3

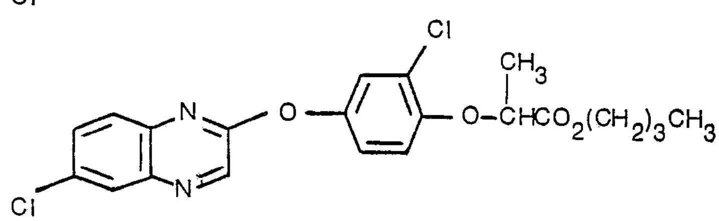

4

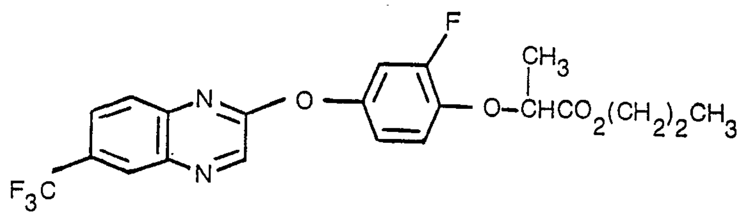

5

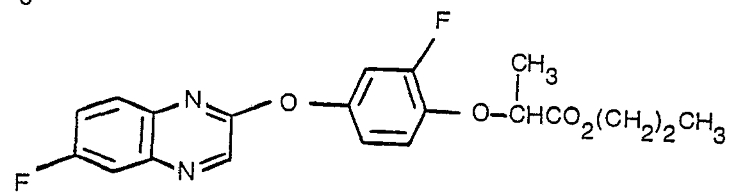

6

Specific examples of compounds of the invention include those detailed in Table 1 below.

TABLE 1

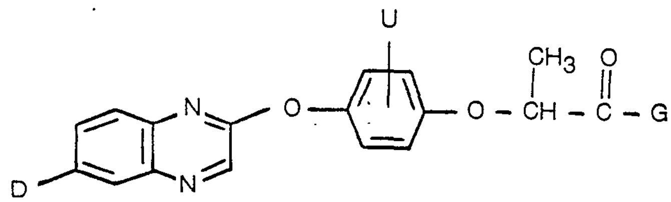

I

| Compound No. | Substituents | | |
|---|---|---|---|
| | D | U | G |
| 1 | Cl | 2-F | $C_2H_5O$ |
| 7 | Cl | 2-F | $CH_3(CH_2)_2O$ |
| 8 | Cl | 2-F | $CH_3(CH_2)_3O$ |
| 9 | Cl | 2-F | HO |
| 10 | Br | 2-F | $C_2H_5O$ |
| 11 | Br | 2-F | $CH_3(CH_2)_2O$ |
| 12 | Br | 2-F | $CH_3(CH_2)_3O$ |
| 13 | Br | 2-F | HO |
| 14 | F | 2-F | $C_2H_5O$ |
| 15 | F | 2-F | $CH_3(CH_2)_2O$ |
| 16 | F | 2-F | $CH_3(CH_2)_3O$ |
| 17 | Cl | 3-F | $C_2H_5O$ |

The compounds of the invention may be prepared by a variety of methods and in a further aspect the invention provides methods for the preparation of the compounds of formula I.

Compounds of formula I wherein G is not hydroxy may be prepared from the acid of formula Ib (I; G=OH) by, for example, neutralisation of the acid with a base to give an acid salt or esterification of the acid with an alcohol or thiol to give an acid ester (SCHEME A). Processes known in the art for the preparation of

acid salts and acid esters may be adapted, without undue experimentation, to prepare compounds of the invention of formula I from compounds of the invention of formula Ib.

SCHEME A

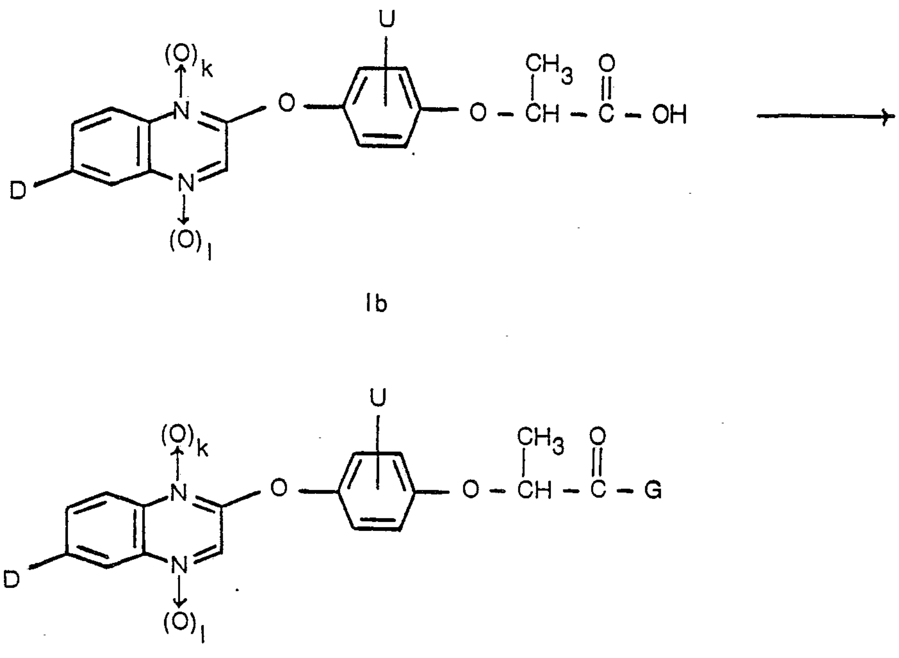

Ib

I

N-oxides of the invention of formula I wherein k and/or l is 1 may be prepared from compounds of the invention of formula I wherein k and/or l is 0 by oxidation. Processes known in the art for the conversion of quinoxalines to quinoxaline N-oxides, for example oxidations using persulfates, peroxides, peracids or peresters, may be adapted without undue experimentation, to prepare N-oxides of the invention.

Compounds of formula I wherein D, U, G, k and l are as hereinbefore defined may be prepared by the condensation of a phenol of formula IX with a compound of formula X wherein hal is chlorine, bromine or iodine, preferably in the presence of an alkaline material; according to SCHEME B.

SCHEME B

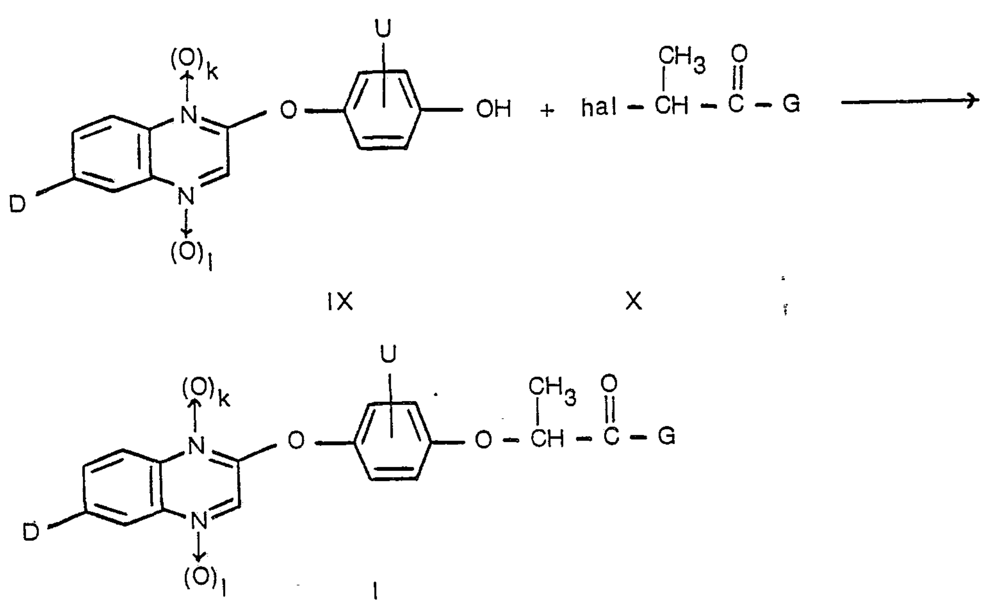

IX X

I

Compounds of formula I may also be prepared by:

a) the condensation of the appropriate quinoxaline derivative of formula V, wherein L is a leaving group (for example, alkylsulfonyl, chlorine, bromine or iodine) with the appropriate phenol of formula VI according to SCHEME C; or

SCHEME C

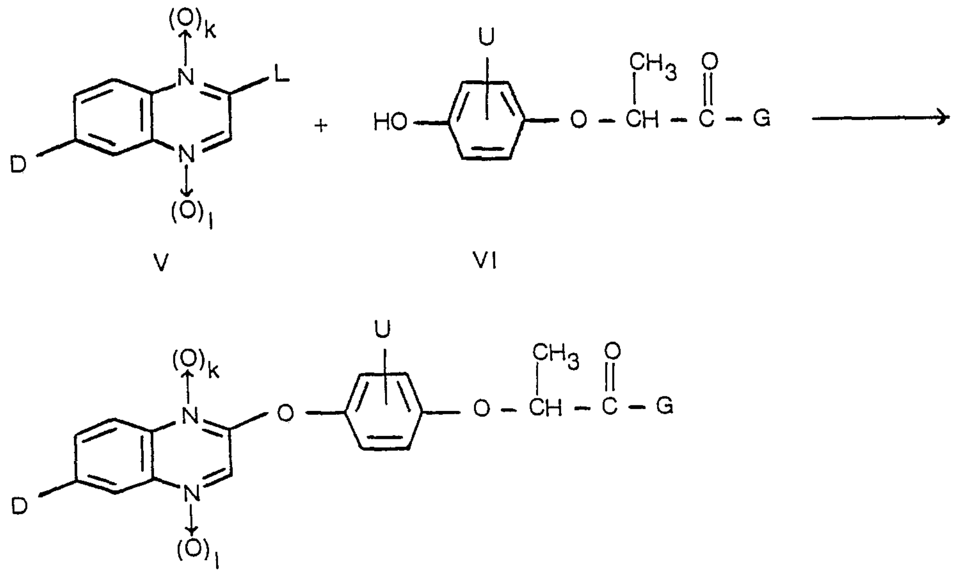

V VI

I

b) the following steps in sequence:

(i) the condensation of the appropriate quinoxaline derivative of formula V, wherein L is a leaving group (for example, alkylsulfonyl, chlorine, bromine or iodine) with the appropriate compound of formula VII, wherein Q is hydroxy or $C_1$ to $C_6$ alkoxy to give a compound of formula VIII wherein Q is hydroxy or $C_1$ to $C_6$ alkoxy;

(ii) the dealkylation of the compound of formula VIII prepared in step (i) above wherein Q is $C_1$ to $C_6$ alkoxy to give a compound of formula IX; and

(iii) the condensation of the product of formula IX obtained in step (i) or step (ii) above with a compound of formula X according to the process described for SCHEME B above (Steps (i) and (ii) are shown in SCHEME D); or

SCHEME D

(i)

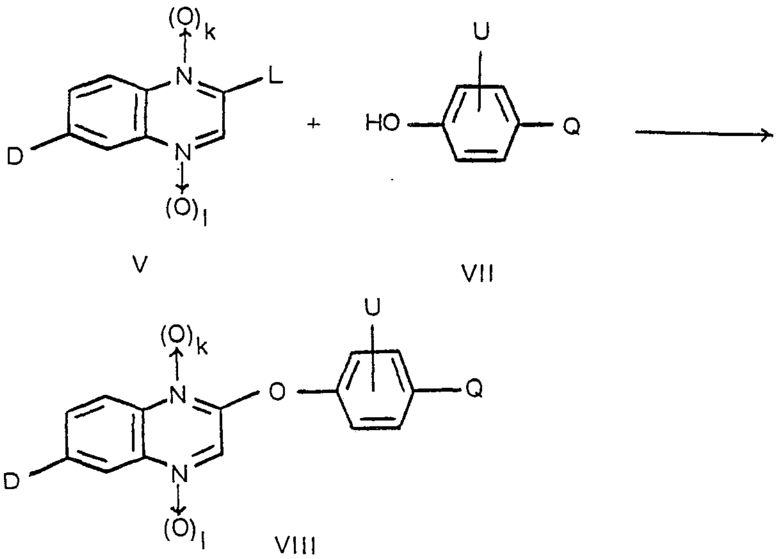

V VII

VIII

SCHEME D (cont.)

(ii)

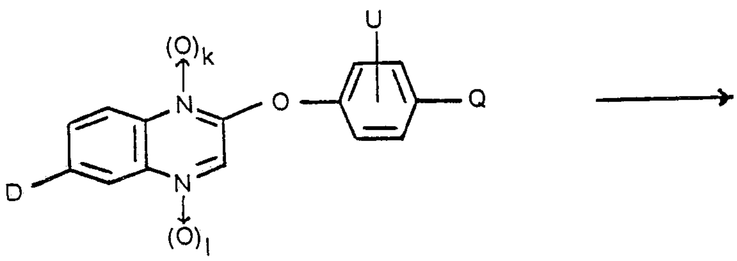

VIII

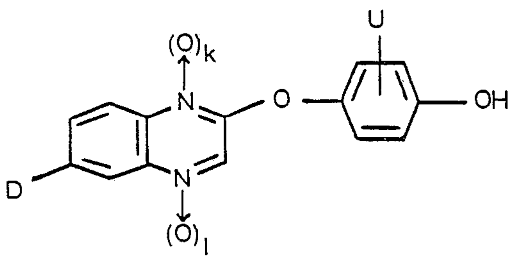

IX

c) the following steps in sequence:

(i) the condensation of the appropriate quinoxaline derivative of formula XI with the appropriate benzene derivative of formula XII wherein L is a leaving group (for example, alkylsulfonyl, chlorine, bromine or iodine) and Q is hydroxy or $C_1$ to $C_6$ alkoxy, to give a compound of formula VIII wherein Q is as hereinbefore defined;

(ii) the dealkylation of the compound of formula VIII prepared in step (i) above wherein Q is $C_1$ to $C_6$ alkoxy to give a compound of formula IX according to the process described for SCHEME D step (ii) above; and

(iii) the condensation of the product of formula IX obtained in step (i) or step (ii) above with a compound of formula X according to the process described for SCHEME B above (step (i) is shown in SCHEME E).

SCHEME E

(i)

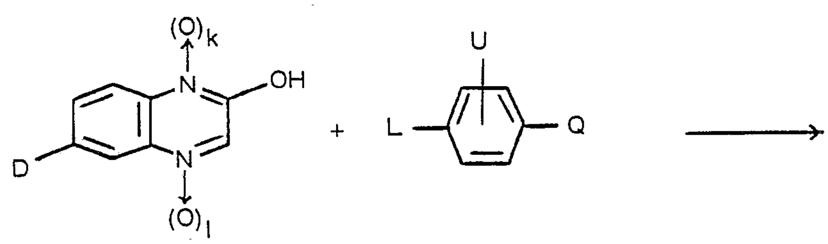

XI XII

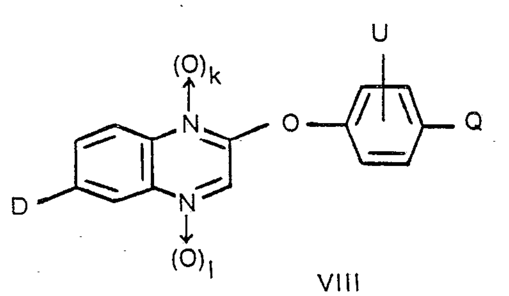

VIII

The condensation reaction illustrated in SCHEMES B to E and outlined above are preferably carried out in the presence of an alkaline material and preferably in the presence of a solvent. Suitable alkaline materials include alkali metal and alkaline earth metal hydroxides and carbonates such as sodium hydroxide, potassium hydroxide, sodium carbonate and potassium carbonate. Suitable solvents include ketones such as, for example, acetone, methyl ethyl ketone and methyl isobutyl ketone, and dipolar aprotic solvents such as, for example, dimethylformamide, dimethylacetamide, dimethylsulfoxide, *N*-methylpyrrolidone, hexamethylphosphoramide and sulfolan.

The reaction conditions required to effect the condensation reactions illustrated in SCHEMES B, C, D, and E and outlined above vary according to the nature of the reactants and the solvent used. In general the reaction is facilitated by the application of heat and usually a reaction temperature in the range of 40° to 150°C and reaction time of between 0.5 and 20 hours is satisfactory. However, higher or lower reaction temperatures and/or shorter or longer reaction times may be used if desired.

The dealkylation reactions illustrated in SCHEME D and outlined in paragraphs b) (ii) and c) (ii) above may be effected using a variety of reagents known in the art. For example, aryl-alkyl ethers may be cleaved using reagents such as pyridine hydrochloride, hydroidic acid, hydrobromic acid, sodium thioethoxide in dimethylformamide, acetyl p-toluene-sulphonate, sodium or potassium iodide in formic or acetic acid, lithium iodide in 2,4,6-collidine and boron tribromide. Reaction times and reaction conditions vary widely depending on the dealkylation agent used and the ether to be cleaved. The reaction conditions generally employed when using the above "ether-cleavage" reagents are known to those skilled in the art and may be adapted without undue experimentation to effect the "ether-cleavage" reactions illustrated in SCHEME D and outlined in paragraph b) (ii) and c) (ii) above.

The compounds of formula VIII

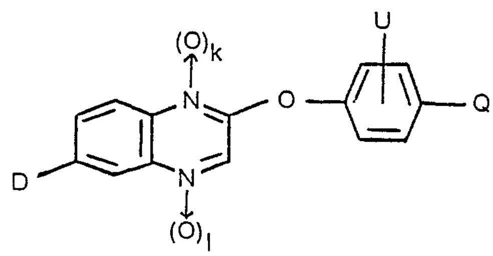

VIII,

which are useful intermediates in the preparation of compounds of formula I, are also believed to be novel compounds. Therefore, in a further embodiment the invention provides compounds of formula VIII wherein D, U, k, l and Q are as hereinbefore defined.

The compounds of formula I are active as herbicides and therefore, in a further aspect the invention provides a process for severely damaging or killing unwanted plants which process comprises applying to the plants, or to the growth medium of the plants, an effective amount of a compound of formula I as hereinbefore defined.

Generally speaking the compounds of formula I are herbicidally effective against a variety of plants. However, certain of the compounds of the invention are selectively active against monocotyledonous plants, dicotyledonous plants being relatively unaffected by rates of application of the compounds of the invention which are severely damaging or lethal to other plant species.

Moreover, certain of the compounds of formula I are selectively active within the group of monocotyledonous plants and may be used at a rate sufficient to kill or severely damage monocotyledonous weeds in a monocotyledonous cereal crop.

Therefore, in yet a further aspect the invention provides a process for selectively controlling the growth of weeds in crops which process comprises applying to the crop, or to the growth medium of the crop, a compound of formula I, as hereinbefore defined, in an amount sufficient to severely damage or kill the weeds but insufficient to damage the crop substantially.

The compounds of formula I may be applied directly to the plant (post-emergence application) or to the soil before the emergence of the plant (pre-emergence application). However, the compounds are, in general, more effective when applied to the plant post-emergence.

The compounds of formula I may be used on their own to exhibit the growth of, severely damage, or kill plants but are preferably used in the form of a composition comprising a compound of the invention in admixture with a carrier comprising a solid or liquid diluent. Therefore, in yet a further aspect the invention provides plant growth inhibiting, plant damaging, or plant killing compositions comprising a compound of formula I as hereinbefore defined and an inert carrier therefor.

Compositions according to the invention include both dilute compositions, which are ready for immediate use, and concentrated compositions, which require to be diluted before use, usually with water. Preferably the compositions contain from 0.01% to 90% by weight of the active ingredient. Dilute compositions ready for use preferably contain from 0.01 to 2% of active ingredient, while concentrated compositions may contain from 20 to 90% of active ingredient, although from 20 to 70% is usually preferred.

The solid compositions may be in the form of granules, or dusting powders wherein the active ingredient is mixed with a finely divided solid diluent, e.g. kaolin, bentonite, kieselguhr, dolomite, calcium carbonate, talc, powdered magnesia, Fuller's earth and gypsum. They may also be in the form of dispersible powders or grains, comprising a wetting agent to facilitate the dispersion of the powder or grains in liquid. Solid compositions in the form of a powder may be applied as foliar dusts.

Liquid compositions may comprise a solution or dispersion of an active ingredient in water optionally containing a surface-active agent, or may comprise a solution or dispersion of an active ingredient in a water-immiscible organic solvent which is dispersed as droplets in water.

Surface-active agents may be of the cationic, anionic, or non-ionic type. The cationic agents are, for example, quaternary ammonium compounds (e.g. cetyltrimethylammonium bromide). Suitable anionic agents are soaps; salts of aliphatic mono esters of sulphuric acid, for example sodium lauryl sulphate; and salts dodecylbenzenesulphonate, sodium, calcium, and ammonium lignosulphonate, butylnaphthalene sulphonate, and a mixture of the sodium salts of diisopropyl- and triisopropylnaphthalenesulphonic acid. Suitable non-ionic agents are the condensation products of ethylene oxide with fatty alcohols such as oleyl alcohol and cetyl alcohol, or with alkylphenols such as octyl- or nonyl-phenol or octyl-cresol. Other non-ionic agents are the partial esters derived from long chain fatty acids and hexitol anhydrides, for example sorbitan monolaurate; the condensation products of the partial ester with ethylene oxide; and the lecithins.

The aqueous solutions or dispersions may be prepared by dissolving the active ingredient in water or an organic solvent optionally containing wetting or dispersing agent(s) and then, when organic solvents are used, adding the mixture so obtained to water optionally containing wetting or dispersing agent(s). Suitable organic solvents include, for example, ethylene dichloride, isopropyl alcohol, propylene glycol, diacetone alcohol, toluene, kerosene, methylnaphthalene, the xylenes and trichloroethylene.

The compositions for use in the form of aqueous solutions or dispersions are generally supplied in the form of a concentrate containing a high proportion of the active ingredient, and the concentrate is then diluted with water before use. The concentrates are usually required to withstand storage for prolonged periods and after such storage, to be capable of dilution with water to form aqueous preparations which remain homogeneous for a sufficient time to enable them to be applied by conventional spray equipment. Concentrates conveniently contain 20—90%, preferably 20—70%, by weight of the active ingredient(s). Dilute preparations ready for use may contain varying amounts of the active ingredient(s) depending upon the intended purpose; amounts of 0.01% to 10.0% and preferably 0.1% to 2%, by weight of active ingredient(s) are normally used.

A preferred form of concentrated composition comprises the active ingredient which has been finely divided and which has been dispersed in water in the presence of a surface-active agent and a suspending agent. Suitable suspending agents are hydrophilic colloids and include, for example, polyvinylpyrrolidone and sodium carboxymethylcellulose, and the vegetable gums, for example gum acacia and gum tragacanth. Preferred suspending agents are those which impart thixotropic properties to, and increase the viscosity of the concentrate. Examples of preferred suspending agents include hydrated colloidal mineral silicates, such as montmorillonite, beidellite, nontronite, hectorite, saponite, and saucorite. Bentonite is especially preferred. Other suspending agents include cellulose derivatives and polyvinyl alcohol.

The rate of application of the compounds of the invention will depend on a number of factors including, for example, the compound chosen for use, the identity of the plants whose growth is to be inhibited, the formulations selected for use and whether the compound is to be applied for foliage or root uptake. As a general guide, however, an application rate of from 0.005 to 20 kilograms per hectare is suitable while from 0.01 to 5 kilograms per hectare may be preferred.

The compositions of the invention may comprise, in addition to one or more compounds of the invention, one or more compounds not of the invention but which possess biological activity. For example, as hereinbefore indicated the compounds of the invention are in general substantially more effective against monocotyledonous plants or grass species than against dicotyledonous plants or broad-leaved species. As a result, in certain applications the herbicidal use of the compounds of the invention alone may be insufficient to protect a crop. Accordingly in yet a still further embodiment the invention provides a herbicidal composition comprising a mixture of at least one herbicidal compound of formula I as hereinbefore defined with at least one other herbicide.

The other herbicide may be any herbicide not having the formula I. It will generally be a herbicide having a complementary action. For example, one preferred class is of mixtures comprising a herbicide active against broad-leaved weeds. A second preferred class is of mixtures comprising a contact herbicide.

Examples of useful complementary herbicides include:

A. benzo-2,1,3-thiadiazin-4-one-2,2-dioxides such as 3-isopropylbenzo-2,1,3-thiadiazin-4-one-2,2-dioxide (common name bentazon);

B. hormone herbicides and in particular the phenoxy-alkanoic acids such as 4-chloro-2-methylphenoxy acetic acid (common name MCPA), 2-(2,4-dichlorophenoxy)propionic acid (common name dichlorprop), 2,4,5-trichlorophenoxyacetic acid (common name 2,4,5-T), 4-(4-chloro-2-methylphenoxy)-butyric acid (common name MCPB), 2,4-dichlorophenoxyacetic acid (common name 2,4-D), 4-(2,4-dichlorophenoxy)butyric acid (common name 2,4-DB), 2-(4-chloro-2-methylphenoxy)propionic acid (common name mecoprop), and their derivatives (e.g. salts, esters, amides and the like);

C. 3-[4-(4-halophenoxy)phenyl]-1,1-dialkylureas such as 3-[4-(4-chlorophenoxy)phenyl]-1,1-dimethylurea (common name chloroxuron);

D. dinitrophenols and their derivatives (e.g. acetates) such as 2-methyl-4,6-dinitrophenol (common name DNOC), 2-tertiarybutyl-4,6-dinitrophenol (common name dinoterb), 2-secondarybutyl-4,6-dinitrophenol (common name dinoseb) and its ester dinoseb acetate;

E. dinitroaniline herbicides such as N',N'-diethyl-2,6-dinitro-4-trifluoromethyl-*m*-phenylenediamine (common name dinitramine), 2,6-dinitro-N,N-dipropyl-4-trifluoromethylaniline (common name trifluralin) and 4-methylsulfonyl-2,6-dinitro-N,N-dipropylaniline (common name nitralin);

F. phenylurea herbicides such as N'-(3,4-dichlorophenyl)-N,N-dimethylurea (common name diuron) and N,N-dimethyl-N'-[3-(trifluoromethyl)phenyl]urea (common name fluometuron);

G. phenylcarbamoyloxyphenylcarbamates such as 3-[(methoxycarbonyl)amino]phenyl (3-methylphenyl)-carbamate (common name phenmedipham) and 3-[(ethoxycarbonyl)amino]phenyl phenylcarbamate (common name desmedipham);

H. 2-phenylpyridazin-3-ones such as 5-amino-4-chloro-2-phenylpyridazin-3-one (common name pyrazon);

I. uracil herbicides such as 3-cyclohexyl-5,6-trimethyleneuracil (common name lenacil), 5-bromo-3-*sec*-butyl-6-methyluracil (common name bromacil) and 3-*tert*-butyl-5-chloro-6-methyluracil (common name terbacil);

J. triazine herbicides such as 2-chloro-4-ethylamino-6-(iso-propylamino)-1,3,5-triazine (common name atrazine), 2-chloro-4,6-di(ethylamino)-1,3,5-triazine (common name simazine) and 2-azido-4-(iso-propylamino)-6-methylthio-1,3,5-triazine (common name aziprotryne);

K. 1-alkoxy-1-alkyl-3-phenylurea herbicides such as 3-(3,4-dichlorophenyl)-1-methoxy-1-methylurea (common name linuron), 3-(4-chlorophenyl)-1-methoxy-1-methylurea (common name monolinuron) and 3-(4-bromo-4-chlorophenyl)-1-methoxy-1-methylurea (common name chlorobromuron);

L. thiolcarbamate herbicides such as S-propyl dipropylthiocarbamate (common name verolate);

M. 1,2,4-triazin-5-one herbicides such as 4-amino-4,5-dihydro-3-methyl-6-phenyl-1,2,4-triazine-5-one (common name metamitron) and 4-amino-6-*tert*-butyl 4,5-dihydro-3-methylthio-1,3,4-triazin-5-one (common name metribuzin);

N. benzoic acid herbicides such as 2,3,6-trichlorobenzoic acid (common name 2,3,6-TBA), 3,6-dichloro-2-methoxybenzoic acid (common name dicamba) and 3-amino-2,5-dichlorobenzoic acid (common name chloramben);

O. anilide herbicides such as N-butoxymethyl-α-chloro-2',6'-diethylacetanilide (common name butachlor), the corresponding N-methoxy compound (common name alachlor), the corresponding N-*iso*-propyl compound (common name propachlor) and 3',4'-dichloropropionanilide (common name propanil);

P. dihalobenzonitrile herbicides such as 2,6-dichlorobenzonitrile (common name dichlorbenil), 3,5-dibromo-4-hydroxybenzonitrile (common name bromoxynil) and 3,5-diiodo-4-hydroxybenzonitrile (common name ioxynil).

Q. haloalkanoic herbicides such as 2,2-dichloropropionic acid (common name dalapon), trichloroacetic acid (common name TCA) and salts thereof;

R. diphenylether herbicides such as 4-nitrophenyl 2-nitro-4-trifluoromethylphenyl ether (common name fluorodifen), methyl 5-(2,4-dichlorophenoxy)-2-nitrobenzoate (common name bifenox), 2-nitro-5-(2-chloro-4-trifluoromethylphenoxy)benzoic acid and 2-chloro-4-trifluoromethylphenyl 3-ethoxy-4-nitrophenyl ether; and

S. miscellaneous herbicides including N,N-dimethyldiphenylacetamide (common name diphenamid), N-(1-naphthyl)phthalamic acid (common name naptalam) and 3-amino-1,2,4-triazole.

Examples of useful contact herbicides include:

T. bipyridylium herbicides such as those in which the active entity is the 1,1'-dimethyl-4,4'-dipyridylium ion (common name paraquat) and those in which the active entity is the 1,1'-ethylene-2,2'-dipyridylium ion (common name diquat);

U. organoarsenical herbicides such as monosodium methanearsonate (common name MSMA); and

V. amino acid herbicides such as N-(phosphonomethyl)-glycine (common name glyphosate) and its salts and esters.

The invention is now illustrated by, but in no way limited to, the following Examples.

## Example 1

*Ethyl 2-[2-Fluoro-4-(6-chloroquinoxalin-2-yloxy)phenoxy]propionate (1)*

a) A solution of potassium persulphate (70 g, 0.26 mole) in water (1.5 l) was added slowly, with stirring, to a solution of 3-fluorophenol (29 g, 0.26 mole) and sodium hydroxide (52 g, 1.3 mole) in water (520 ml) so that the temperature of the mixture remained below 20°C. The dark solution was stirred overnight at room temperature and then acidified to Congo red with hydrochloric acid. The solution was then extracted with diethyl ether (3 × 200 ml) to remove unreacted phenol. The aqueous layer was made neutral and evaporated to dryness and the residue was extracted into 90% ethanol (700 ml) to give a solution of 2-fluoro-4-hydroxyphenyl potassium sulphate. The solution was concentrated to 250 ml, sodium hydroxide (12 g, 0.3 mole) in water (50 ml) was added and then the solution was boiled and stirred while benzyl chloride (35 g, 0.28 mole) was added dropwise. Heating was continued for a further 2 hours and then

concentrated hydrochloric acid was added to Congo red and the mixture boiled for 2 hours. The mixture was concentrated under reduced pressure then diluted with water and extracted with ether. The ether extracts were dried ($MgSO_4$) and evaporated to give the crude product. The product was purified by chromatography on silica gel with methylene chloride elution to give 2-fluoro-4-benzyloxyphenol (12.5 g, 25%), m.p. 67°C.

A mixture of 2-fluoro-4-benzyloxyphenol (11.85 g, 0.054 mole), ethyl 2-bromopropionate (9.83 g, 0.054 mole), anhydrous potassium carbonate (8.20 g, 0.059 mole) and ethyl methyl ketone (200 ml) was stirred and refluxed for 3 hours. The solvent was removed on a rotary evaporator and the residue partitioned between methylene chloride and water. The organic layer was dried ($MgSO_4$) and evaporated to give a pale brown oil (15.5 g) which slowly solidified. Recrystallization from ethanol gave the product, ethyl 2-[2-fluoro-4-(benzyloxy)phenoxy]-propionate, as nearly colourless crystals (13.0 g; 75%), m.p. 63°C.

10% Palladium on charcoal (1.5 g) was added to a solution of ethyl 2-(2-fluoro-4-benzyloxyphenoxy) propionate (13 g) in ethanol (250 ml) and the stirred suspension was placed under hydrogen at atmospheric pressure for 2 hours. The catalyst was removed by filtration and the filtrate evaporated under reduced pressure to give the product, ethyl 2-(2-fluoro-4-hydroxyphenoxy)propionate, as a colourless oil (8.9 g, 95%). Proton magnetic resonance spectrum ($CDCl_3$, δ in ppm): 1.3 t, 3H ($CH_2C\underline{H}$); 1.6, d, 3H ($-CHC\underline{H}_3$); 4.25, q, 2H ($OC\underline{H}_2CH_3$); 4.7, q, 1H ($-C\underline{H}CH_3$); 6.4—7.1, m, 4H (O$\underline{H}$ and phenoxy ring).

b) A mixture of 2,6-dichloroquinoxaline (0.99 g 0.005 mole), ethyl 2-(2-fluoro-4-hydroxyphenoxy)propionate (1.14 g, 0.005 mole), anhydrous potassium carbonate (0.76 g, 0.0055 mole) and dimethylformamide (20 ml) was stirred and heated at 100°C for 2 hours. The mixture was cooled and then poured into water (200 ml) to give a white precipitate which was collected by filtration. Recrystallization from ethanol gave the product, ethyl 2-[2-fluoro-4-(6-chloroquinoxalin-2-yloxy)phenoxy]propionate, as colourless crystals (1.3 g; 66%) m.p. 110°C. Proton magnetic resonance spectrum ($CDCl_3$, δ in ppm); 1.3, t, 3H ($CH_2C\underline{H}_3$); 1.7, d, 3H ($CHC\underline{H}_3$); 4.3 q, 2H ($OC\underline{H}_2CH_3$); 4.8, q, 1H ($C\underline{H}CH_3$); 6.9—7.5, m, 3H (phenoxy ring); 7.8 bs, 2H (C7, C8 of quinoxaline); 8.2, bs, 1H (C5 of quinoxaline); 8.8, s, 1H (C3 of quinoxaline).

## Example 2

*Ethyl 2-[2-fluoro-4-(6-bromoquinoxalin-2-yloxy)phenoxy]propionate* (10) was prepared from 6-bromo-2-chloroquinoxaline and ethyl 2-(2-fluoro-4-hydroxyphenoxy)propionate following essentially the same procedure as that described in Example 1 part (b). The title compound was obtained as colourless crystals m.p. 107—108.6°C and the assigned structure was characterised by nuclear magnetic resonance spectroscopy.

## Example 3

*2-[2-Fluoro-4-(6-chloroquinoxalin-2-yloxy)phenoxy]propionic acid* (9)

Ethyl 2-[2-fluoro-4-(6-chloroquinoxalin-2-yloxy)phenoxy]propionate (3.23 g; 8.27 mmole) was dissolved in a warm mixture of isopropanol (150 ml) and tetrahydrofuran (30 ml). The solution was allowed to cool to a temperature of 25°C and a solution of sodium hydroxide (0.34 g) in water (10 ml) was added slowly with stirring. The reaction mixture was stirred at room temperature for a period of 10 minutes and then aqueous 2M sodium hydroxide (0.5 ml) was added and the solution was stirred at room temperature for 5 hours. The solvent was removed from the reaction mixture by evaporation under reduced pressure and the residue was dissolved in water. The aqueous solution was treated with aqueous 2M hydrochloric acid until acidic and the precipitated product was collected by filtration and air dried. The product was recrystallised from toluene to give the title compound (1.83 g; 61%), m.p. 132—138°C. The assigned structure was confirmed by nuclear magnetic resonance spectroscopy.

## Example 4

*2-[2-Fluoro-4-(6-bromoquinoxalin-2-yloxy)phenoxy]propionic acid* (13) was prepared from the corresponding ethyl ester by hydrolysis following essentially the same procedure as that described in Example 3. The assigned structure was confirmed by nuclear magnetic resonance spectroscopy.

## Example 5

*n-Propyl 2-[2-fluoro-4-(6-chloroquinoxalin-2-yloxy)phenoxy]propionate* (7)

a) 2-[2-Fluoro-4-(6-chloroquinoxalin-2-yloxy)phenoxy]propionic acid (1.73 g; 4.77 mmole) was treated with thionyl chloride and the mixture was heated under reflux with stirring for a period of 5 hours. The excess thionyl chloride was removed by distillation under reduced pressure to give 2-[2-fluoro-4-(6-chloroquinoxalin-2-yloxy)phenoxy]propionyl chloride as a straw coloured oil.

b) 2-[2-Fluoro-4-(6-chloroquinoxalin-2-yloxy)phenoxy]propionyl chloride (0.9 g; 2.3 mmole) was dissolved in dry dichloromethane and the solution was added slowly with stirring to a cooled mixture of *n*-propanol (5 ml) and triethylamine (1 ml). The mixture was stirred at room temperature for a period of 4 hours and then the solvent was evaporated under vacuum and the residue was dissolved in dichloromethane. The solution was washed with water and the organic phase was dried over magnesium sulfate. The solution was filtered and the solvent was removed under vacuum to give a light brown oil (0.92 g; 95.3%). The product was crystallised from ethanol to give the title compound as a crystalline solid (0.58 g; 60.4%); m.p. 70—72°C. The assigned structure was confirmed by nuclear magnetic resonance spectroscopy.

Example 6

The following compounds were prepared from the appropriate acid and the appropriate alcohol following essentially the same procedure as that described in Example 5:

*n-butyl 2-[2-fluoro-4-(6-chloroquinoxalin-2-yloxy)phenoxy]propionate* (8), solid, m.p. 56—60.5°C;
*n-propyl 2-[2-fluoro-4-(6-bromoquinoxalin-2-yloxy)phenoxy]propionate* (11), solid, m.p. 63—64.5°C; and
*n-butyl 2-[2-fluoro-4-(6-bromoquinoxalin-2-yloxy)phenoxy]propionate* (12), oil

Each of the assigned structures was confirmed by nuclear magnetic resonance spectroscopy.

Example 7

*n-Propyl 2-[2-fluoro-4-(6-fluoroquinoxalin-2-yloxy)phenoxy]propionate* (15)

a)(i) A solution of potassium persulfate (70.0 g) in water (1.5 l) was added slowly with stirring to a solution of 2-fluorophenol (29.0 g) and sodium hydroxide (52.0 g) in water (520 ml) the reaction mixture being maintained at a temperature of 10 to 20°C throughout the addition. The reaction mixture was stirred overnight at room temperature and then acidified with concentrated hydrochloric acid to Congo Red. The aqueous solution was extracted with diethyl ether (to remove any unreacted phenol) and then neutralized by the addition of an aqueous saturated solution of sodium hydrogen carbonate. The neutralized aqueous solution was evaporated to dryness to give a brown residue which was triturated with 90% ethanol (several times using 2 l of ethanol overall). The ethanolic extracts were filtered and the solvent evaporated to give an orange solid (58.0 g).

The solid was dissolved in a solution comprising a mixture of 90% ethanol (300 ml), water (100 ml) and sodium hydroxide (9.9 g). The mixture was heated to reflux and ethyl α-bromopropionate (44.8 g) was slowly added. The reaction mixture was heated under reflux for a period of 4½ hours. The mixture was cooled to 60—70°C, acidified to Congo Red with concentrated hydrochloric acid and then heated under reflux for a further 2½ hours. The solution was cooled and the solvent was evaporated under vacuum to give a brown solid. The solid was treated with water and the aqueous mixture was extracted several times with diethyl ether. The combined etherial extracts were washed with water, dried over anhydrous magnesium sulfate, filtered and the ether was evaporated to give a brown oil (30.0 g).

The brown oil was dissolved in ethanol (150 ml), a solution of sodium hydroxide (5.3 g) in water (50 ml) was added and the mixture was stirred at a temperature of 50°C for a period of 4 hours. The solvent was removed from the reaction mixture under vacuum and the residue was acidified with dilute hydrochloric acid. The mixture was extracted several times with diethyl ether and the combined etherial extracts were washed with water, dried over anhydrous magnesium sulfate, filtered, and the solvent removed under reduced pressure to give crude 2-(2-fluoro-4-hydroxyphenoxy)propionic acid (20.5 g; 39.6%).

a)(ii) n-Propanol (35 ml) and concentrated sulfuric acid (6 drops) were added to a solution of crude 2-(2-fluoro-4-hydroxyphenoxy)propionic acid (8.6 g) in dichloroethane (13 ml). The mixture was heated under reflux with stirring for a period of 6.5 hours, cooled, and the solvent was evaporated under reduced pressure. The residue was treated with water and the aqueous mixture was extracted with dichloromethane. The organic phase was washed with a saturated aqueous solution of sodium hydrogen carbonate and then with water. The organic phase was dried over anhydrous magnesium sulfate, filtered and the solvent was evaporated under reduced pressure to give an oil (5.7 g; 54.8%. The oil was purified by vacuum distillation to give *n*-propyl 2-(2-fluoro-4-hydroxyphenoxy)propionate (3.7 g; 35.5%); bp 210—220°C at 0.1 mmHg.

b) A mixture of 2-chloro-6-fluoroquinoxaline (1.0 g; 5.48 mmole), *n*-propyl 2-(2-fluoro-4-hydroxyphenoxy)propionate (1.33 g; 5.49 mmole), anhydrous potassium carbonate (0.8 g) and anhydrous dimethylformamide (40 ml) was stirred and heated at a temperature of 120°C for a period of 3.5 hours. The solvent was evaporated from the reaction mixture under vacuum, the residue was treated with water and the mixture was extracted with diethyl ether. The etherial extract was washed with water, dried over anhydrous magnesium sulfate, filtered, and the solvent was evaporated under vacuum to give a brown oil. The product was purified by preparative thin layer chromatography over silica gel (eluant chloroform/ethanol, 95:5) to give the title compound (0.89 g; 41.8%) as a solid; m.p. 49—51°C.

The assigned structure was confirmed by nuclear magnetic resonance spectroscopy and mass spectrometry.

Example 8

The following compounds were prepared from the appropriate alkyl 2-(2-fluoro-4-hydroxyphenoxy)propionate, prepared from the appropriate alcohol and 2-(2-fluoro-4-hydroxyphenoxy)propionic acid according to the procedure described in Example 7 part (a)(ii), and 2-chloro-6-fluoroquinoxaline following essentially the same procedure as that described in Example 7 part (b):

*ethyl 2-[2-fluoro-4-(6-fluoroquinoxalin-2-yloxy)phenoxy]propionate* (14), solid, m.p. 58—59°C; and
*n-butyl 2-[2-fluoro-4-(6-fluoroquinoxalin-2-yloxy)phenoxy]propionate* (16), solid; m.p. 57—58°C.

Each of the assigned structures was confirmed by nuclear magnetic resonance spectroscopy.

## Example 9

*Ethyl 2-[3-fluoro-4-(6-chloroquinoxalin-2-yloxy)phenoxy]*propionate (17) was prepared from 2-fluorophenol following the same procedure as that described in Example 1.

The first step yielded 3-fluoro-4-benzyloxyphenol as a colourless solid, m.p. 80°C.

Ethyl 2-(3-fluoro-4-benzyloxyphenoxy)propionate was obtained as colourless crystals, m.p. 77°C.

Ethyl 2-(3-fluoro-4-hydroxyphenoxy)propionate was obtained as a colourless oil. Proton magnetic resonance spectrum ($CDCl_3$; δ in ppm): 1.25 (t, 3H); 1.55 (d, 3H); 4.25 (q, 2H); 4.7 (q, 1H); 6.15 (br. s, 1H); 6.45—7.1 (m, 3H).

The title compound, ethyl 2-[3-fluoro-4-(6-chloroquinoxalin-2-yloxy)phenoxy]propionate was obtained as colourless crystals, m.p. 109°C.

## Example 10

Concentrated formulations of the compounds of the invention were prepared by:

a) in the case of oils and waxy solids, dissolving the compound in toluene containing 7% v/v "Teric" N13 ("Teric" is a Trade Mark and "Teric" N13, a product of ethoxylation of nonylphenol, is available from ICI Australia Limited) and 3% v/v "Kemmat" SC15B ("Kemmat" is a Trade Mark and "Kemmat" SC15B is a formulation of calcium dodecylbenzene sulfonate; or

b) in the case of crystalline solids, adding 5 parts by weight of the compound and 1 part by weight of "Dyapol" PT ("Dyapol" is a Trade Mark and "Dyapol" PT is an anionic suspending agent) to 94 parts by weight of an aqueous solution containing 0.25% v/v of "Teric" N8 (a product of ethoxylation of nonylphenol) and ball-milling the mixture to produce a stable suspension. The emulsifiable concentrates and suspensions were then diluted with water to give an aqueous composition of the required concentration suitable for use in the evaluation of the pre-emergence and post-emergence herbicidal activity of the compounds of the invention.

## Example 11

The pre-emergent herbicidal activity of the compounds of the invention formulated as described in Example 10 was assessed by the following procedure.

The seeds of the test species were sown in rows 2 cm deep in soil contained in seed boxes. The monocotyledonous plants and the dicotyledonous plants were sown in separate boxes and after sowing the two boxes were sprayed with the required quantity of a composition of the invention. Two duplicate seed boxes were prepared in the same manner but were not sprayed with a composition of the invention and were used for comparison purposes. All the boxes were placed in a glasshouse, lightly watered with an overhead spray to initiate germination and then sub-irrigated as required for optimum plant growth. After three weeks the boxes were removed from the glasshouse and the effect of the treatment was visually assessed. The results are presented in Table 2 where the damage to plants is rated on a scale of from 0 to 3 where 0 represents from 0 to 25% damage, 3 represents 75 to 99% kill and 3+ represents 100% kill. A dash (—) means that no experiment was carried out.

The names of the test plants are as follows :

| | |
|---|---|
| Wh | Wheat |
| Ot | Wild oats |
| Rg | Ryegrass |
| Jm | Japanese millet |
| P | Peas |
| Ip | Ipomea |
| Ms | Mustard |
| Sf | Sunflower |

TABLE 2

PRE-EMERGENCE HERBICIDAL ACTIVITY

| Compound No. | Application Rate kg/ha | Test Plant | | | | | | | |
|---|---|---|---|---|---|---|---|---|---|
| | | Wh | Ot | Rg | Jm | P | Ip | Ms | Sf |
| 1 | 1.0 | 3 | 3 | 3+ | 3+ | 0 | 0 | 0 | 0 |
| 1 | 0.5 | 1 | 3 | 3 | 3+ | 0 | 0 | 0 | 0 |
| 1 | 0.25 | 0 | 1 | 3 | 3+ | 0 | 0 | 0 | 0 |
| 17 | 5.0 | 2 | 1 | 3+ | 3+ | 0 | 0 | 0 | 0 |
| 17 | 1.0 | 1 | 0 | 3 | 3 | 0 | 0 | 0 | 0 |

Example 12

The post-emergent herbicidal activity of the compounds of the invention formulated as described in Example 10 was assessed by the following procedure.

The seeds of the test species were sown in rows 2 cm deep in soil contained in seed boxes. The monocotyledonous plants and the dicotyledonous plants were sown in separate seed boxes in duplicate. The four seed boxes were placed in a glasshouse, lightly watered with an overhead spray to initiate germination and then sub-irrigated as required for optimum plant growth. After the plants had grown to a height of about 10 to 12.5 cm one box of each of the monocotyledonous plants and the dicotyledonous plants was removed from the glasshouse and sprayed with the required quantity of a composition of the invention. After spraying the boxes were returned to the glasshouse for a further 3 weeks and the effect of treatment was visually assessed by comparison with the untreated controls. The results are presented in Table 3 where the damage to plants is rated on a scale of from 0 to 3 where 0 represents 0 to 25% damage, 3 represents 75 to 99% kill and 3+ represents 100% kill. A dash (—) means that no experiment was carried out.

The names of the test plants are as follows:

| | |
|---|---|
| Wh | Wheat |
| Ot | Wild Oats |
| Rg | Ryegrass |
| Jm | Japanese millet |
| P | Peas |
| Ip | Ipomea |
| Ms | Mustard |
| Sf | Sunflower |

TABLE 3
POST-EMERGENCE HERBICIDAL ACTIVITY

| Compound No. | Application Rate kg/ha | Test Plant | | | | | | | |
|---|---|---|---|---|---|---|---|---|---|
| | | Wh | Ot | Rg | Jm | P | Ip | Ms | Sf |
| 1 | 1.0 | 3+ | 3+ | 3+ | 3+ | 0 | 0 | 0 | 0 |
| 1 | 0.5 | 3+ | 3+ | 3+ | 3+ | 0 | 0 | 0 | 0 |
| 1 | 0.25 | 3 | 3+ | 3+ | 3+ | 0 | 0 | 0 | 0 |
| 17 | 5.0 | 3 | 2 | 3+ | 3+ | 0 | 0 | 0 | 0 |
| 17 | 1.0 | 2 | — | 3 | 3+ | 0 | 0 | 0 | 0 |

Example 13

The compounds were formulated for test by mixing an appropriate amount with 5 ml of an emulsion prepared by diluting 160 ml of a solution containing 21.8 g per litre of "Span" 80 and 78.2 g per litre of "Tween" 20 in methylcyclohexanone to 500 ml with water. "Span" 80 is a Trade Mark for a surface-active agent comprising sorbitan monlaurate. "Tween" 20 is a Trade Mark for a surface-active agent comprising a condensate of sorbitan monolaurate with 20 molar proportions of ethylene oxide. Each 5 ml emulsion containing a test compound was then diluted to 40 ml with water and sprayed on to youngrpot plants (post-emergence test) of the species named in Table 4 below. Damage to test plants was assessed after 14 days on a scale of 0 to 5 where 0 is 0 to 20% damage and 5 is complete kill. In a test for pre-emergence herbicidal activity, seeds of the test plants were sown in a shallow slit formed in the surface of soil in fibre trays. The surface was then levelled and sprayed, and fresh soil then spread thinly over the sprayed surface. Assessment of herbicidal damage was carried out after 21 days using the same scale of 0 to 5 as the post-emergence test. In both cases the degree of herbicidal damage was assessed by comparison with untreated control plants. The results are given in Table 4 below. A dash (—) means that no experiment was carried out.

The names of the test plants are as follows:

| | |
|---|---|
| Sb | Sugar beet |
| Rp | Rape |
| Ct | Cotton |
| Sy | Soy bean |
| Mz | Maize |
| Mw | Winter wheat |
| Rc | Rice |
| Sn | *Senecio vulgaris* |
| Ip | *Ipomea purpurea* |
| Am | *Amaranthus retroflexus* |
| Pi | *Polygonum aviculare* |
| Ca | *Chenopodium album* |
| Ga | *Galium aparine* |
| Xa | *Xanthium pensylvanicum* |

| | |
|---|---|
| Ab | *Abutilon theophrasti* |
| Co | *Cassia Obtusifolia* |
| Av | *Avena fatua* |
| Dg | *Digitaria sanguinalis* |
| Al | *Alopecurus myosuroides* |
| St | *Setaria viridis* |
| Ec | *Echinochloa crus-galli* |
| Sh | *Sorghum halepense* |
| Ag | *Agropyron repens* |
| Cn | *Cyperus rotundas* |

TABLE 4 – PART A

| Compound No. | APPLICATION Method Rate (kg/ha) | | TEST PLANT | | | | | | | | | | | |
|---|---|---|---|---|---|---|---|---|---|---|---|---|---|---|
| | | | Sb | Rp | Ct | Sy | Mz | Ww | Rc | Sn | Ip | Am | Pi | Ca |
| 1 | PRE | 0.2 | 0 | 0 | 0 | 0 | 2 | 4 | 5 | 0 | 0 | 0 | – | 0 |
| 1 | PRE | 0.05 | 0 | 0 | 0 | 0 | 0 | 3 | 0 | 0 | 0 | 1 | – | 0 |
| 1 | PRE | 0.01 | – | – | – | – | 0 | 1 | 0 | – | – | – | – | – |
| 1 | POST | 0.2 | 1 | 2 | 0 | 1 | 4 | 4 | 5 | 1 | 0 | 1 | – | – |
| 1 | POST | 0.05 | 0 | 0 | 0 | 0 | 5 | 4 | 4 | 0 | 0 | 0 | – | – |
| 1 | POST | 0.01 | – | – | – | – | 4 | 4 | 0 | – | – | – | – | – |
| 10 | FRE | 0.025 | 0 | 0 | 0 | – | 1 | 3 | 2 | 1 | 0 | – | – | 0 |
| 10 | PRE | 0.01 | 0 | 0 | 0 | 0 | 0 | 1 | 0 | 0 | 0 | – | 0 | 0 |
| 10 | POST | 0.025 | 0 | 0 | 0 | 1 | 4 | 4 | 0 | J | 0 | – | 0 | 0 |
| 10 | POST | 0.01 | 0 | 0 | 0 | 1 | 2 | 3 | 0 | 0 | 0 | – | 0 | 0 |
| 14 | PRE | 0.02 | – | – | – | – | 0 | 3 | 3 | – | – | – | – | – |
| 14 | PRE | 0.01 | – | – | – | – | 1 | 1 | 1 | – | – | – | – | – |
| 14 | POST | 0.02 | – | – | – | – | 4 | 3 | 2 | – | – | – | – | – |
| 14 | POST | 0.01 | – | – | – | – | 4 | 3 | 1 | – | – | – | – | – |
| 17 | PRE | 2.0 | 0 | 0 | 0 | 0 | 4 | 5 | 5 | 0 | 0 | 0 | – | 0 |
| 17 | PRE | 0.5 | – | – | – | – | 4 | 4 | 4 | – | – | – | – | – |
| 17 | PRE | 0.05 | – | – | – | – | 1 | 0 | 0 | – | – | – | – | – |
| 17 | POST | 2.0 | 1 | 0 | 0 | 2 | 5 | 4 | 2 | 0 | 0 | 0 | – | – |
| 17 | POST | 0.5 | – | – | – | – | 5 | 4 | 4 | – | – | – | – | – |
| 17 | POST | 0.05 | – | – | – | – | 5 | 2 | 0 | – | – | – | – | – |

TABLE 4 – PART B

| Compound No. | APPLICATION Method Rate (kg/ha) | TEST PLANT | | | | | | | | | | | |
|---|---|---|---|---|---|---|---|---|---|---|---|---|---|
| | | Ga | Xa | Ab | Co | Av | Dg | Al | St | Ec | Sh | Ag | Cn |
| 1 | PRE 0.2 | – | 0 | 0 | 0 | 4 | 3 | 4 | 4 | 3 | 3 | 5 | 1 |
| 1 | PRE 0.05 | – | 0 | 0 | 0 | 1 | 0 | 3 | 1 | 0 | 0 | 4 | 0 |
| 1 | PRE 0.01 | – | – | – | – | 0 | 0 | 0 | 0 | 0 | 1 | 1 | 0 |
| 1 | POST 0.2 | 0 | 0 | 0 | 0 | 5 | 5 | 5 | 5 | 5 | 5 | 4 | 0 |
| 1 | POST 0.05 | 0 | 0 | 0 | 0 | 4 | 5 | 5 | 5 | 5 | 5 | 3 | 0 |
| 1 | POST 0.01 | – | – | – | – | 4 | 3 | 3 | 4 | 4 | 4 | 2 | 1 |
| 10 | PRE 0.025 | 0 | 0 | 0 | 0 | 0 | 0 | 2 | 0 | 0 | 0 | 4 | 0 |
| 10 | PRE 0.01 | 0 | 0 | 0 | 0 | 0 | 0 | 0 | 0 | 0 | 0 | 4 | 0 |
| 10 | POST 0.025 | – | – | – | – | 4 | 4 | 4 | 5 | 5 | 3 | 5 | 0 |
| 10 | POST 0.01 | – | – | – | – | 3 | 4 | 3 | 2 | 5 | 2 | 0 | 0 |
| 14 | PRE 0.02 | – | – | – | – | 0 | 2 | 2 | 0 | 1 | 0 | 2 | 0 |
| 14 | PRE 0.01 | – | – | – | – | 0 | 0 | 1 | 0 | 0 | 0 | 1 | 0 |
| 14 | POST 0.02 | – | – | – | – | 4 | 4 | 4 | 4 | 5 | 5 | 2 | 0 |
| 14 | POST 0.01 | – | – | – | – | 4 | 3 | 4 | 3 | 5 | 5 | 0 | 0 |
| 17 | PRE 2.0 | – | 0 | 0 | 0 | 3 | 4 | 5 | 5 | 5 | 5 | 5 | 1 |
| 17 | PRE 0.5 | – | – | – | – | 3 | 5 | 5 | 5 | 5 | 4 | 5 | 2 |
| 17 | PRE 0.05 | – | – | – | – | 1 | 3 | 3 | 0 | 0 | 3 | 3 | 0 |
| 17 | POST 2.0 | 0 | 0 | 0 | 0 | 3 | 5 | 5 | 5 | 5 | 5 | 4 | 0 |
| 17 | POST 0.5 | – | – | – | – | 2 | 4 | 3 | 4 | 5 | 5 | 2 | 0 |
| 17 | POST 0.05 | – | – | – | – | 1 | 1 | 2 | 3 | 3 | 1 | 1 | 0 |

Example 14

This Example illustrates the selective herbicidal activity of the compounds of the invention.

The compounds were formulated and applied to the test species following the procedure described in Example 13. The species of test plant and the results are given in Tables 5 and 6 below. Damage to the test plants was assessed 26 days after treatment on a scale of 0 to 9 where 0 represents 0 to 10% damage and 9 represents 90 to 100% damage. A dash (—) means that no experiment was carried out.

TABLE 5

| Compound No. | APPLICATION Method Rate (kg/ha) | | TEST PLANT | | | | | | | |
|---|---|---|---|---|---|---|---|---|---|---|
| | | | Crops | | | | | Weeds | | |
| | | | Pe | Rp | Sb | Lt | Av | Al | Bt | Ag |
| 1 | PRE | 0.4 | 1 | 0 | 0 | 2 | 2 | 8 | 6 | 8 |
| 1 | PRE | 0.2 | 0 | 0 | 0 | 1 | 2 | 5 | 1 | 3 |
| 1 | POST | 0.4 | 0 | 0 | 0 | – | 6 | 4 | 5 | 2 |
| 1 | POST | 0.2 | 0 | 0 | 0 | – | 3 | 3 | 2 | 2 |
| 7 | PRE | 0.4 | 0 | 0 | 0 | 0 | 6 | 9 | 4 | 9 |
| 7 | PRE | 0.2 | 0 | 0 | 0 | 0 | 6 | 7 | 2 | 6 |
| 14 | PRE | 0.4 | 0 | 0 | 0 | 0 | 8 | 9 | 7 | 9 |
| 14 | PRE | 0.2 | 0 | 0 | 0 | 0 | 9 | 9 | 8 | 9 |

The names of the Test Plants are as follows:

Pe Pea
Rp Rape
Sb Sugar beet
Lt Lettuce
Av *Avena fatua*
Al *Alopecurus myosuroides*
Bt *Bromus tectorum*
Ag *Agropyron repens*

TABLE 6

| Compound No. | APPLICATION Method Rate (kg/ha) | TEST PLANT Crops Sy | Ct | To | Weeds Ec | Dg | St | Sh | Pm |
|---|---|---|---|---|---|---|---|---|---|
| 1 | PRE 0.1 | 0 | – | 0 | 4 | 7 | 1 | 9 | 1 |
| 1 | PRE 0.05 | – | – | 0 | 7 | 9 | 4 | 8 | 5 |
| 1 | PRE 0.025 | 0 | 0 | 0 | 8 | 9 | 9 | 9 | 9 |
| 1 | POST 0.1 | 0 | 0 | 0 | 9 | 9 | 9 | 9 | 9 |
| 1 | POST 0.05 | 0 | 0 | 0 | 9 | 9 | 9 | 9 | 9 |
| 1 | POST 0.025 | 0 | 0 | 0 | 9 | 9 | 8 | 9 | 9 |
| 7 | PRE 0.2 | – | – | – | 9 | 9 | 9 | 7 | 9 |
| 7 | PRE 0.1 | 0 | 0 | 0 | 9 | 8 | 7 | 5 | 9 |
| 7 | PRE 0.05 | 0 | 1 | 0 | 8 | 7 | 6 | 3 | 8 |
| 14 | PRE 0.2 | 0 | 1 | 0 | 9 | 8 | 9 | 8 | 9 |
| 14 | PRE 0.1 | 0 | 0 | 0 | 9 | 8 | 9 | 6 | 9 |
| 14 | PRE 0.05 | 0 | 0 | 0 | 9 | 8 | 8 | 4 | 9 |

The names of the Test Plants are as follows:

Sy Soyabean
Ct Cotton
To Tomato
Ec *Echinochloa crus-galli*
Dg *Digitaria sanguinalis*
St *Setaria viridis*
Sh *Sorghum halepense*
Pm *Panicum maximum*

**Description for the Contracting States: AT BE CH DE FR GB IT LI NL SE**

The description for the Contracting State LU serves also as the description for all other designated Contracting States except that the definition of the compound of formula I is subject to the proviso that when D is chloro, k and I are 0 and U is fluoro in the 3-position, then G is not ethoxy. As a consequence Compound 17 of Table I does not form a part of the description for any Contracting State except LU.

**Claims for the Contracting States: BE CH DE FR GB IT LI NL SE**

1. A compound of formula I:

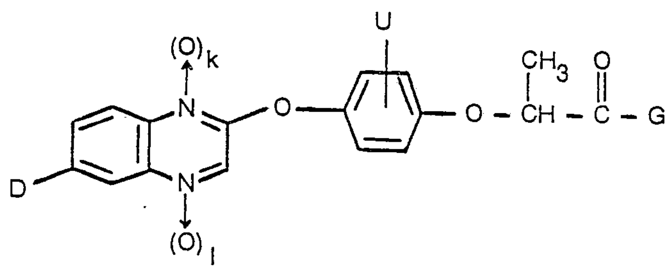

I

or a salt thereof wherein:

D and U are independently chosen from halogen, methyl and halomethyl;

G is chosen from hydroxy, mercapto, $C_1$ to $C_{10}$ alkoxy, $C_2$ to $C_{10}$ alkenyloxy, $C_3$ to $C_{10}$ alkynyloxy, $C_1$ to $C_{10}$ alkylthio, $C_2$ to $C_{10}$ alkenylthio, $C_3$ to $C_{10}$ alkynylthio, $C_3$ to $C_7$ cycloalkoxy, and the group OM wherein M is an alkyl metal or an alkaline earth metal ion; and

k and l are independently chosen from 0 to 1;

subject to the proviso that when D is chloro, k and l are O and U is fluoro in the 3-position, G is not ethoxy.

2. A compound according to claim 1 wherein:

D is chosen from fluorine, chlorine, bromine, iodine and trifluoromethyl;

U is chosen from fluorine, chlorine, bromine and iodine substituted in the 2-position of the benzene ring;

G is chosen from hydroxy, $C_1$ to $C_6$ alkoxy, $C_2$ to $C_6$ alkenyloxy, $C_3$ to $C_6$ alkynyloxy, cyclohexyloxy and the group OM wherein M is an alkali metal ion; and

k and l are both 0.

3. A compound according to claim 1 or 2 wherein:

D is chosen from fluorine, chlorine and bromine;

U is fluorine substituted in the 2-position of the benzene ring;

G is chosen from hydroxy and $C_1$ to $C_6$ alkoxy; and

k and l are both 0.

4. A compound according to any one of claims 1 to 3 wherein:

D is chosen from fluorine and chlorine;

U is fluorine substituted in the 2-position of the benzene ring;

G is chosen from hydroxy, ethoxy, *n*-propoxy and *n*-butoxy; and

k and l are both 0.

5. The compound *n*-propyl 2-[2-fluoro-4-(6-chloroquinoxalin-2-yloxy)phenoxy]propionate.

6. The compound *n*-propyl 2-[2-fluoro-4-(6-fluoroquinoxalin-2-yloxy)phenoxy]propionate.

7. A composition having herbicidal activity against monocotyledonous weeds in dicotyledonous crops characterised by consisting essentially of an enantiomer of a compound of formula I or a salt thereof as claimed in any one of claims 1 to 6, said composition being substantially free from the other enantiomer.

8. A herbicidal composition comprising as active ingredient a compound as claimed in any one of claims 1 to 6 and a carrier therefor.

9. A herbicidal composition comprising, as active ingredients,

(a) a compound of formula I or a salt thereof as claimed in any one of claims 1 to 6; and

(b) another herbicide which is a benzo-2,1,3-thiadiazin-4-one-2,2-dioxide; a hormone herbicide; a 3-[4-(4-halophenoxy)phenyl]-1,1-dialkyl urea; a dinitrophenol or a derivative thereof; a dinitroaniline herbicide; a phenylurea herbicide; a phenylcarbamoyloxyphenylcarbamate; a 2-phenylpyridazin-3-one; a uracil herbicide; a triazine herbicide; a 1-alkoxy-1-alkyl-3-phenylurea herbicide; a thiolcarbamate herbicide; a 1,2,4-triazin-5-one herbicide; a benzoic acid herbicide; an anilide herbicide; a dihalobenzonitrile herbicide; a haloalkanoic acid herbicide or a salt thereof; a diphenyl ether herbicide; N,N-dimethyldiphenylacetamide; N(1-naphthyl)phthalamic acid; 3-amino-1,2,4-triazole; a bipyridylium herbicide; an organoarsenical herbicide; or an amino acid herbicide or a salt or ester thereof.

10. A process for severely damaging or killing unwanted plants which process comprises applying to said plants, or to the growth medium of said plants, an effective amount of a compound as claimed in any one of claims 1 to 6 or an effective amount of a composition as claimed in any one of claims 7 to 9.

11. A process for selectively controlling the growth of monocotyledonous weeds in dicotyledonous crops which process comprises applying to said crop, or to the growth medium of said crop, a compound as claimed in any one of claims 1 to 6 or a composition as claimed in any one of claims 7 to 9 in an amount sufficient to severely damage or kill the weeds but insufficient to substantially damage the crop.

12. A process according to claim 10 or claim 11 wherein the compound is applied at a rate in the range from 0.005 to 20 kilograms per hectare.

13. A process for the synthesis of a compound of formula I as claimed in any one of claims 1 to 6 which process comprises either the reaction of a quinoxaline derivative of formula IX with a compound of the formula X:

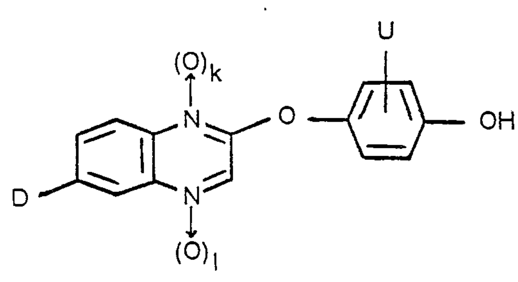

IX

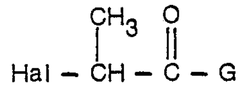

X

wherein D, U, G, k and l are as defined in any one of claims 1 to 4 and hal is chlorine, bromine or iodine; or the reaction of a quinoxaline derivative of formula X with a compound of formula VI:

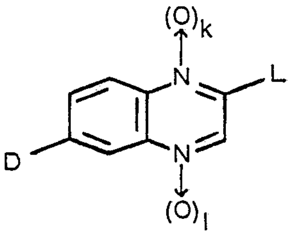

+

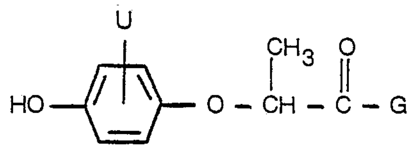

V VI

wherein D, U, G, k and l are as defined in any one of claims 1 to 4 and L is a leaving group.

14. A process according to claim 13 wherein the quinoxaline compound of formula IX is prepared by reacting a quinoxaline derivative of formula V as defined in claim 13, with a phenol of formula VII:

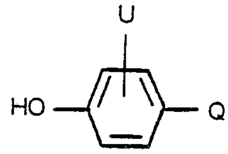

VII

wherein U is as defined in any one of claims 1 to 4 and Q is hydroxy or $C_1$ to $C_6$ alkoxy, to give a compound of formula VIII

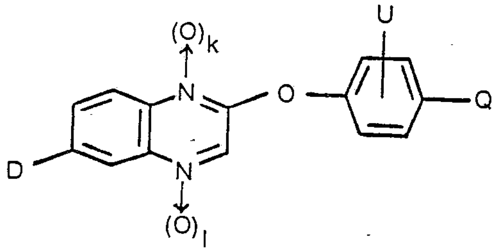

VIII

wherein D, U, k and l are as defined in any one of claims 1 to 4 and Q is as defined above, and when Q is $C_1$ to $C_6$ alkoxy, dealkylating the compound of formula VIII to give a compound of formula IX as defined in claim 13.

15. A process for the synthesis of a compound of formula I or a salt thereof as claimed in any one of claims 1 to 6 which comprises dealkylating a compound of formula VIII:

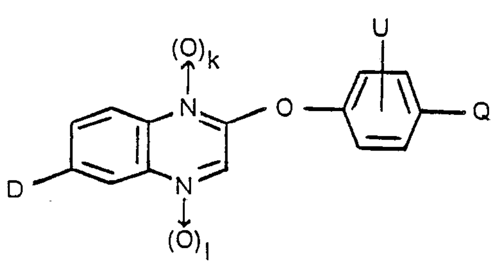

VIII

wherein D, U, k and l are as defined in any one of claims 1 to 4 and Q is $C_1$ to $C_6$ alkoxy, to give a compound of formula IX:

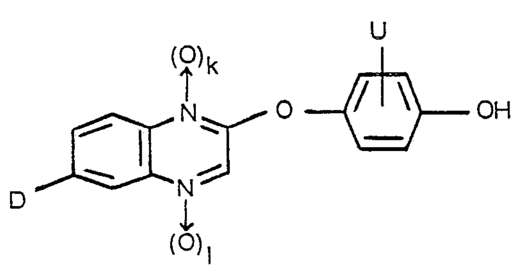

IX

and then reacting the compound of formula IX with a compound of formula X:

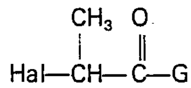

X

wherein G is as defined in any one of claims 1 to 4 and hal is chlorine, bromine or iodine.

16. A compound of formula VIII:

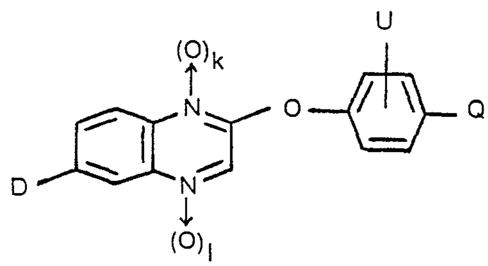

VIII

wherein D, U, k and l are as defined in any one of claims 1 to 4 and Q is hydroxy or $C_1$ to $C_6$ alkoxy.

**Claims for the Contracting State: AT**

1. A process for the synthesis of a compound of formula I:

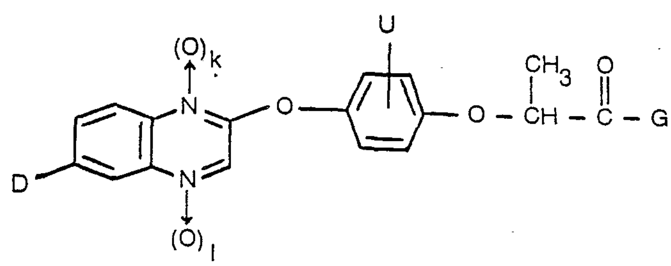

I

or a salt thereof wherein:

D and U are independently chosen from halogen, methyl and halomethyl;

G is chosen from hydroxy, mercapto, $C_1$ to $C_{10}$ alkoxy $C_1$ to $C_{10}$ alkenyloxy, $C_3$ to $C_{10}$ alkynyloxy, $C_1$ to $C_{10}$ alkylthio, $C_2$ to $C_{10}$ alkenylthio, $C_3$ to $C_{10}$ alkynylthio, $C_3$ to $C_7$ cycloalkoxy, and the group OM wherein M is an alkyl metal or an alkaline earth metal ion; and

k and l are independently chosen from 0 to 1;

subject to the proviso that when D is chloro, k and l are O and U is fluoro in the 3-position, G is not ethoxy; which process comprises either

(a) reacting a quinoxaline derivative of formula IX:

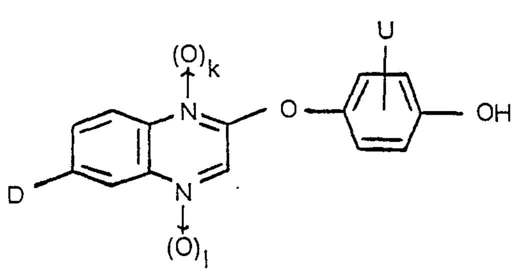

IX

wherein D, U, k and l are as defined above, with a compound of formula X:

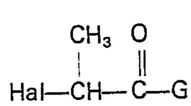

X

wherein G is as defined above and hal is chlorine, bromine or iodine; or

(b) reacting a quinoxaline derivative of formula V:

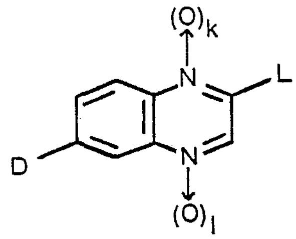

V

wherein D, k and l are as defined above and L is a leaving group, with a compound of formula VI:

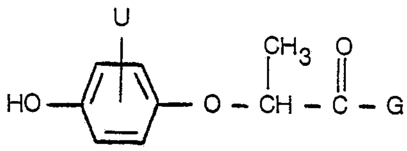

VI

wherein U and G are as defined above.

2. A process according to claim 1 wherein the quinoxaline compound of formula IX is prepared by reacting a quinoxaline derivative of formula V as defined in claim 1, with a phenol of formula VII:

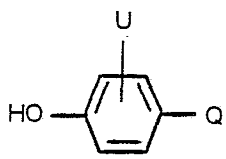

VII

wherein U is as defined in claim 1 and Q is hydroxy or $C_1$ to $C_6$ alkoxy, to give a compound of formula VIII:

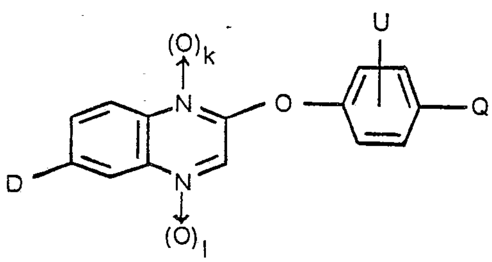

VIII

wherein D, U, k and l are as defined in claim 1 and Q is as defined above, and when Q is $C_1$ to $C_6$ alkoxy, dealkylating the compound of formula VIII to give a compound of formula IX as defined in claim 1.

3. A process according to claim 1 or 2 for the synthesis of a compound of formula I or a salt thereof as defined in claim 1 which comprises dealkylating a compound of formula VIII:

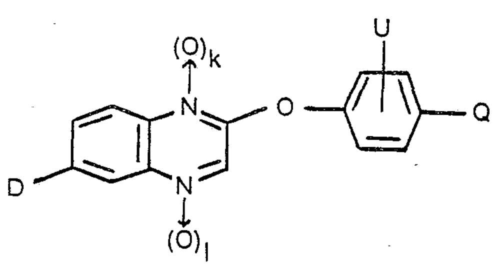

VIII

wherein D, U, k and l are as defined in claim 1 and Q is $C_1$ to $C_6$ alkoxy, to give a compound of formula IX:

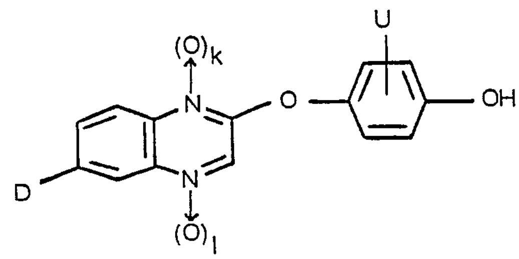

IX

and then reacting the compound of formula IX with a compound of formula X:

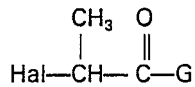

X

wherein G is as defined in claim 1 and hal is chlorine, bromine or iodine.

4. A process according to claims 1, 2 or 3 wherein:

D is chosen from fluorine, chlorine, bromine, iodine and trifluoromethyl;

U is chosen from fluorine, chlorine, bromine and iodine substituted in the 2-position of the benzene ring;

G is chosen from hydroxy, $C_1$ to $C_6$ alkoxy, $C_2$ to $C_6$ alkenyloxy, $C_3$ to $C_6$ alkynyloxy, cyclohexyloxy and the group OM wherein M is an alkali metal ion; and

k and l are both 0.

5. A process according to any one of the preceding claims wherein:

D is chosen from fluorine, chlorine and bromine;

U is fluorine substituted in the 2-position of the benzene ring;

G is chosen from hydroxy and $C_1$ to $C_6$ alkoxy; and

k and l are both 0.

6. A process according to any one of the preceding claims wherein:

D is chosen from fluorine and chlorine;

U is fluorine substituted in the 2-position of the benzene ring;

G is chosen from hydroxy, ethoxy, *n*-propoxy and *n*-butoxy; and

k and l are both 0.

7. A process according to any one of the preceding claims which the reactants are such that *n*-propyl 2-[2-fluoro-4-(6-chloroquinoxalin-2-yloxy)phenoxy]propionate is produced.

8. A process according to any one of the preceding claims wherein the reactants are such that *n*-propyl 2-[2-fluoro-4-(6-fluoroquinoxalin-2-yloxy)phenoxy]propionate is produced.

9. A process according to any one of the preceding claims wherein a compound of formula I or a salt thereof is produced in the form of an enantiomer having herbicidal activity, in the substantial absence of the other enantiomer.

10. A herbicidal composition comprising as active ingredient, a compound of formula I as defined in any one of the preceding claims and a carrier therefor.

11. A herbicidal composition comprising, as active ingredients,

(a) a compound of formula I or a salt thereof as defined in any one of claims 1 to 9; and

(b) another herbicide which is a benzo-2,1,3-thiadiazin-4-one-2,2-dioxide; a hormone herbicide; a 3-[4-(4-halophenoxy)phenyl]-1,1-dialkyl urea; a dinitrophenol or a derivative thereof; a dinitroaniline herbicide; a phenylurea herbicide; a phenylcarbamoyloxyphenylcarbamate; a 2-phenylpyridazin-3-one; a uracil herbicide; a triazine herbicide; a 1-alkoxy-1-alkyl-3-phenylurea herbicide; a thiolcarbamate herbicide; a 1,2,4-triazin-5-one herbicide; a benzoic acid herbicide; an anilide herbicide; a dihalobenzonitrile herbicide; a haloalkanoic acid herbicide or a salt thereof; a diphenyl ether herbicide; N,N-dimethyldiphenylacetamide; N(1-naphthyl)phthalamic acid; 3-amino-1,2,4-triazole; a bipyridylium herbicide; an organoarsenical herbicide; or an amino acid herbicide or a salt or ester thereof.

12. A process for severely damaging or killing unwanted plants which process comprises applying to said plants, or to the growth medium of said plants, an effective amount of a compound as defined in any one of claims 1 to 9 or an effective amount of a composition as defined in claim 10 or 11.

13. A process for selectively controlling the growth of monocotyledonous weeds in dicotyledonous crops which process comprises applying to said crop, or to the growth medium of said crop, a compound as defined in any one of claims 1 to 9 or a composition as claimed in claim 10 or 11 in an amount sufficient to severely damage or kill the weeds but insufficient to substantially damage the crop.

14. A process according to claim 12 or claim 13 wherein the compound is applied at a rate in the range from 0.005 to 20 kilograms per hectare.

**Claims for the Contracting State: LU**

1. A compound of formula I:

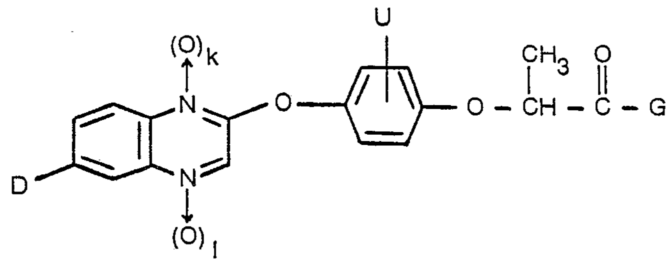

I

or a salt thereof wherein:

D and U are independently chosen from halogen, methyl and halomethyl;

G is chosen from hydroxy, mercapto, $C_1$ to $C_{10}$ alkoxy, $C_1$ to $C_{10}$ alkenyloxy, $C_3$ to $C_{10}$ alkynyloxy, $C_1$ to $C_{10}$ alkylthio, $C_2$ to $C_{10}$ alkenylthio, $C_3$ to $C_{10}$ alkynylthio, $C_3$ to $C_7$ cycloalkoxy, and the group OM wherein M is an alkyl metal or an alkaline earth metal ion; and

k and l are independently chosen from 0 and 1;

2. A compound according to claim 1 wherein:

D is chosen from fluorine, chlorine, bromine, iodine and trifluoromethyl;

U is chosen from fluorine, chlorine, bromine and iodine substituted in the 2-position of the benzene ring;

G is chosen from hydroxy, $C_1$ to $C_6$ alkoxy, $C_2$ to $C_6$ alkenyloxy, $C_3$ to $C_6$ alkynyloxy, cyclohexyloxy and the group OM wherein M is an alkali metal ion; and

k and l are both 0.

3. A compound according to claim 1 or 2 wherein:

D is chosen from fluorine, chlorine and bromine;

U is fluorine substituted in the 2-position of the benzene ring;

G is chosen from hydroxy and $C_1$ to $C_6$ alkoxy; and

k and l are both 0.

4. A compound according to any one of claims 1 to 3 wherein:

D is chosen from fluorine and chlorine;

U is fluorine substituted in the 2-position of the benzene ring;

G is chosen from hydroxy, ethoxy, *n*-propoxy and *n*-butoxy; and

k and l are both 0.

5. The compound *n*-propyl 2-[2-fluoro-4-(6-chloroquinoxalin-2-yloxy)phenoxy]propionate.

6. The compound *n*-propyl 2-[2-fluoro-4-(6-fluoroquinoxalin-2-yloxy)phenoxy]propionate.

7. A composition having herbicidal activity against monocotyledonous weeds in dicotyledonous crops characterised by consisting essentially of an enantiomer of a compound of formula I or a salt thereof as claimed in any one of claims 1 to 6, said composition being substantially free from the other enantiomer.

8. A herbicidal composition comprising as active ingredient a compound as claimed in any one of claims 1 to 6 and a carrier therefor.

9. A herbicidal composition comprising, as active ingredients,

(a) a compound of formula I or a salt thereof as claimed in any one of claims 1 to 6; and

(b) another herbicide which is a benzo-2,1,3-thiadiazin-4-one-2,2-dioxide; a hormone herbicide; a 3-[4-(4-halophenoxy)phenyl]-1,1-dialkyl urea; a dinitrophenol or a derivative thereof; a dinitroaniline herbicide; a phenylurea herbicide; a phenylcarbamoyloxyphenylcarbamate; a 2-phenylpyridazin-3-one; a uracil herbicide; a triazine herbicide; a 1-alkoxy-1-alkyl-3-phenylurea herbicide; a thiolcarbamate herbicide; a 1,2,4-triazin-5-one herbicide; a benzoic acid herbicide; an anilide herbicide; a dihalobenzonitrile herbicide; a haloalkanoic acid herbicide or a salt thereof; a diphenyl ether herbicide; N,N-dimethyldiphenylacetamide; N(1-naphthyl)phthalamic acid; 3-amino-1,2,4-triazole; a bipyridylium herbicide; an organoarsenical herbicide; or an amino acid herbicide or a salt or ester thereof.

10. A process for severely damaging or killing unwanted plants which process comprises applying to said plants, or to the growth medium of said plants, an effective amount of a compound as claimed in any one of claims 1 to 6 or an effective amount of a composition as claimed in any one of claims 7 to 9.

11. A process for selectively controlling the growth of monocotyledonous weeds in dicotyledonous crops which process comprises applying to said crop, or to the growth medium of said crop, a compound as claimed in any one of claims 1 to 6 or a composition as claimed in any one of claims 7 to 9 in an amount sufficient to severely damage or kill the weeds but insufficient to substantially damage the crop.

12. A process according to claim 10 or claim 11 wherein the compound is applied at a rate in the range from 0.005 to 20 kilograms per hectare.

13. A process for the synthesis of a compound of formula I as claimed in any one of claims 1 to 6 which

process comprises either the reaction of a quinoxaline derivative of formula IX with a compound of the formula X:

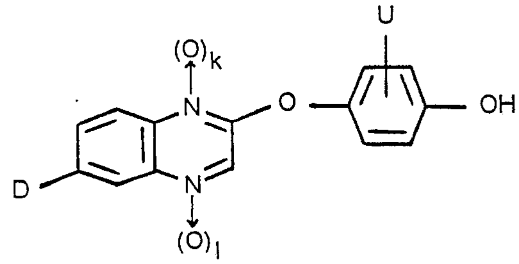

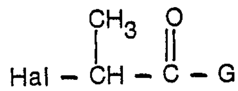

IX X

wherein D, U, G, k and l are as defined in any one of claims 1 to 4 and hal is chlorine, bromine or iodine; or the reaction of a quinoxaline derivative of formula V with a compound of formula VI:

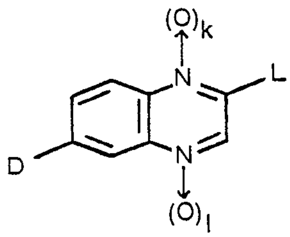

\+

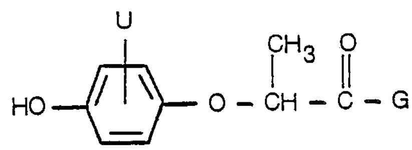

V VI

wherein D, U, G, k and l are as defined in any one of claims 1 to 4 and L is a leaving group.

14. A process according to claim 13 wherein the quinoxaline compound of formula IX is prepared by reacting a quinoxaline derivative of formula V as defined in claim 13, with a phenol of formula VII:

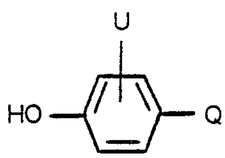

VII

wherein U is as defined in any one of claims 1 to 4 and Q is hydroxy or $C_1$ to $C_6$ alkoxy, to give a compound of formula VIII

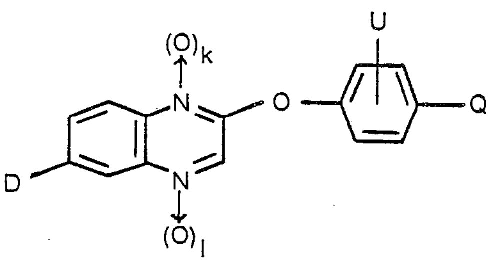

VIII

wherein D, U, k and l are as defined in any one of claims 1 to 4 and Q is as defined above, and when Q is $C_1$ to $C_6$ alkoxy, dealkylating the compound of formula VIII to give a compound of formula IX as defined in claim 13.

15. A process for the synthesis of a compound of formula I or a salt thereof as claimed in any one of claims 1 to 6 which comprises dealkylating a compound of formula VIII:

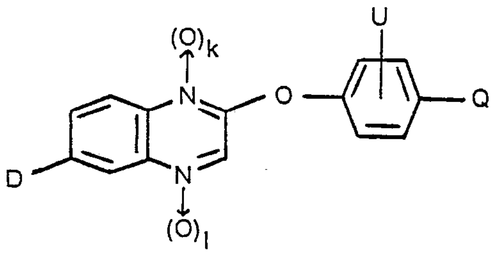

VIII

wherein D, U, k and l are as defined in any one of claims 1 to 4 and Q is $C_1$ to $C_6$ alkoxy, to give a compound of formula IX:

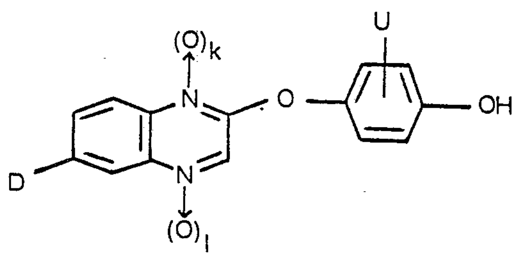

IX

and then reacting the compound of formula IX with a compound of formula X:

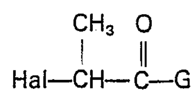

X

wherein G is as defined in any one of claims 1 to 4 and hal is chlorine, bromine or iodine.

16. A compound of formula VIII:

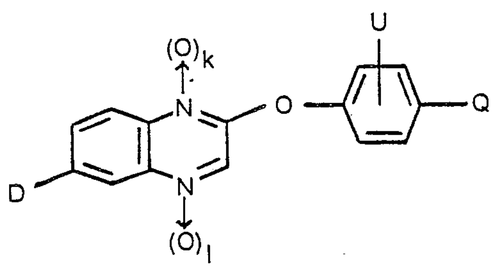

VIII

wherein D, U, k and l are as defined in any one of claims 1 to 4 and Q is hydroxy or $C_1$ to $C_6$ alkoxy.

**Patentansprüche für die Vertragsstaaten: BE CH DE FR GB IT LI NL SE**

1. Verbindung der Formel I

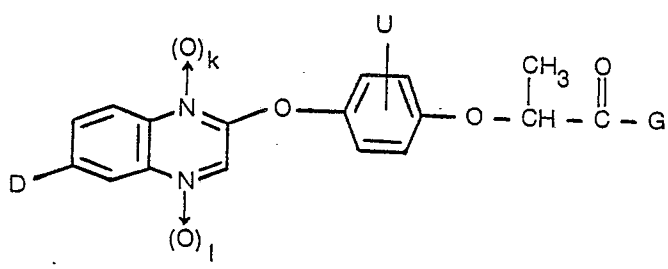

I

oder ein Salz davon, worin

D und U unabhängig ausgewählt sind aus Halogen, Methyl und Halogenomethyl,

G ausgewählt ist aus Hydroxy, Mercapto, $C_1$—$C_{10}$-Alkoxy, $C_2$—$C_{10}$-Alkenyloxy, $C_3$—$C_{10}$-Alkinyloxy, $C_1$—$C_{10}$-Alkylthio, $C_2$—$C_{10}$-Alkenylthio, $C_3$—$C_{10}$-Alkinylthio, $C_3$—$C_7$-Cycloalkoxy under der Gruppe OM, worin M ein Alkalimetall- oder Erdalkalimetallion bedeutet, und

k und l unabhängig ausgewählt sind aus 0 und 1;

mit der Maßgabe, daß G nicht für Äthoxy steht, wenn D für Chloro steht, k und 1 für O stephen und U für Fluoro in der 3-Stellung steht.

2. Verbindung nach Anspruch 1, worin

D ausgewählt ist aus Fluor, Chlor, Brom, Jod und Trifluoromethyl,

U ausgewählt ist aus Fluor, Chlor, Brom und Jod, das in der 2-Stellung des Benzolrings gebunden ist,

G ausgewählt ist aus Hydroxy, $C_1$—$C_6$-Alkoxy, $C_2$—$C_6$-Alkenyloxy, $C_3$—$C_6$-Alkinyloxy, Cyclohexyloxy und der Gruppe OM, worin M ein Alkalimetallion bedeutet, und

k und l beide für O stehen.

3. Verbindung nach Anspruch 1 oder 2, worin

D ausgewählt ist aus Fluor, Chlor und Brom,

U für Fluor steht, das in der 2-Stellung des Benzolrings gebunden ist,

G ausgewählt ist aus Hydroxy und $C_1$—$C_6$ Alkoxy und

k und l beide für O stehen.

4. Verbindung nach einem der Ansprüche 1 bis 3, worin D ausgewählt ist aus Fluor und Chlor,

U für Fluor steht, das in der 2-Stellung des Benzolrings gebunden ist,

G ausgewählt ist aus Hydroxy, Äthoxy, n-Propoxy und n-Butoxy und

k und l beide für O stehen.

5. Die Verbindung 2 - [2 - Fluoro - 4 - (6 - chlorochinoxalin - 2 - yl oxy)phenoxy]propionsäure - n - propylester.

6. Die Verbindung 2 - [2 - Fluoro - 4 - (6 - fluorochinoxalin - 2 - yl oxy)phenoxy]propionsäure - n - propylester.

7. Zusammensetzung mit herbicider Aktivität gegen einkeimblättrige Unkräuter in zweikeimblättrigen Anpflanzungen, dadurch gekennzeichnet, daß sie im wesentlichen aus einem Enantiomer einer Verbindung der Formel I oder einem Salz davon nach einem der Ansprüche 1 bis 6 besteht, wobei die Zusammensetzung im wesentlichen frei vom anderen Enantiomer ist.

8. Herbicide Zusammensetzung, welche als aktiven Bestandteil eine Verbindung nach einem der Ansprüche 1 bis 6 und einen Träger hierfür enthält.

9. Herbicide Zusammensetzung, welche als aktive Bestandteile folgendes enthält:

(a) eine Verbindung der Formel I oder ein Salz davon nach einem der Ansprüche 1 bis 6 und

(b) ein weiteres Herbicid, bei welchem es sich um ein Benzo-2,1,3-thiadiazin-4-on-2,2-dioxid, ein Hormonherbicid, einen 3-[4-(4-Halogenophenoxy)phenyl]-1,1-dialkylharnstoff, ein Dinitrophenol oder ein Derivat davon, ein Dinitroanilinherbicid, ein Phenylharnstoffherbicid, ein Phenylcarbamoyloxyphenylcarbamat, ein 2-Phenylpyridazin-3-on, ein Uracilherbicid, ein Triazinherbicid, ein 1-Alkoxy-1-alkyl-3-phenylharnstoffherbicid, ein Thiolcarbamatherbicid, ein 1,2,4-Triazin-5-on-herbicid, ein Benzoesäureherbicid, ein Anilidherbicid, ein Dihalogenobenzonitrilherbicid, ein Halogenoalkansäureherbicid oder ein Salz davon, ein Diphenylätherherbicid, N,N-Dimethyldiphenylacetamid, N(1-Naphthyl)phthalamidsäure, 3-Amino-1,2,4-triazol, ein Bipyridyliumherbicid, ein Organoarsenherbicid oder ein Aminosäureherbicid oder ein Salz oder einen Ester davon handelt.

10. Verfahren zum starken Schädigen oder Abtöten von unerwünschten Pflanzen, bei welchem auf die Pflanzen oder auf das Wachstumsmedium der Pflanzen eine wirksame Menge einer Verbindung nach einem der Ansprüche 1 bis 6 oder eine wirksame Menge einer Zusammensetzung nach einem der Ansprüche 7 bis 9 aufgebracht wird.

11. Verfahren zur selektiven Bekämpfung des Wachstums von einkeimblättrigen Unkräutern in zweikeimblättrigen Anpflanzungen, bei welchem auf die Anpflanzung oder auf das Wachstumsmedium der Anpflanzung eine Verbindung nach einem der Ansprüche 1 bis 6 oder eine Zusammensetzung nach einem der Ansprüche 7 bis 9 in einer Menge aufgebracht wird, die ausreicht, die Unkräuter stark zu schädigen oder abzutöten, die aber nicht ausreicht, die Anpflanzung wesentlich zu schädigen.

12. Verfahren nach Anspruch 10 oder 11, bei welchem die Verbindung in einer Rate im Bereich von 0,005 bis 20 kg/ha aufgebracht wird.

13. Verfahren zur Synthese einer Verbindung der Formel I nach einem der Ansprüche 1 bis 6, bei welchem entweder ein Chinoxalinderivat der Formel IX mit einer Verbindung der Formel X

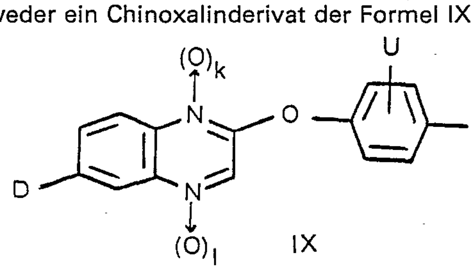

IX

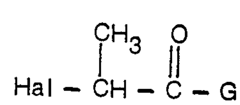

X

worin D, U, G, k und l die in einer der Ansprüche 1 bis 4 angegebenen Bedeutungen besitzen und Hal für Chlor, Brom oder Jod steht, umgesetzt wird oder ein Chinoxalinderivat der Formel V mit einer Verbindung der Formel VI

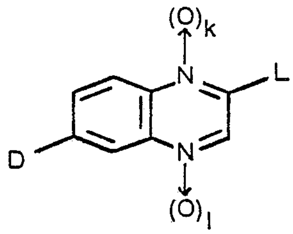

+

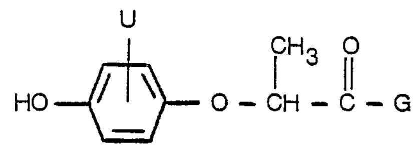

V VI

worin D, U, G, k und l die einem der Ansprüche 1 bis 4 angegebenen Bedeutungen besitzen und L für eine abspaltbare Gruppe steht, umgesetzt wird.

14. Verfahren nach Anspruch 13, bei welchem die Chinoxalinverbindung der Formel IX durch Umsetzung eines Chinoxalinderivats der Formel V nach Anspruch 13 mit einem Phenol der Formel VII

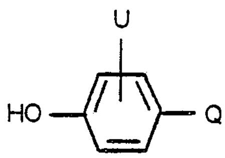

VII

worin U die in einem der Ansprüche 1 bis 4 angegebene Bedeutung besitzt und Q für Hydroxy oder $C_1$—$C_6$-Alkoxy steht, umgesetzt wird, wobei eine Verbindung der Formel VIII

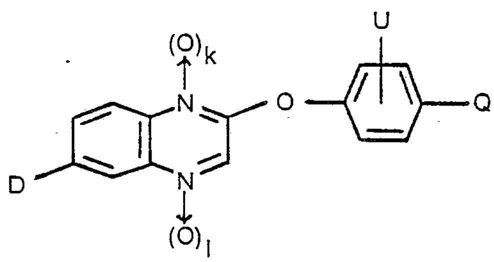

VIII

worin D, U, k und l die in einem der Ansprüche 1 bis 4 angegebenen Bedeutungen besitzen und Q die oben angegebene Bedeutung besitzt, erhalten wird, worauf, wenn Q für $C_1$—$C_6$-Alkoxy steht, die Verbindung der Formel VIII dealkyliert wird, wobei eine Verbindung der Formel IX nach Anspruch 13 erhalten wird.

15. Verfahren zur Synthese einer Verbindung der Formel I oder eines Salzes davon nach einem der Ansprüche 1 bis 6, bei welchem eine Verbindung der Formel VIII

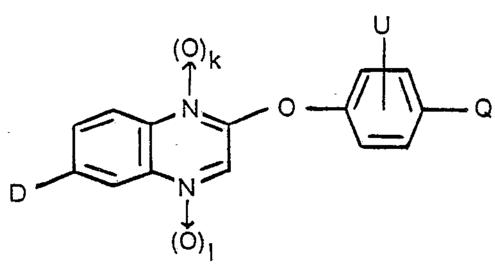

VIII

worin D, U, k und l die in einem der Ansprüche 1 bis 4 angegebenen Bedeutungen besitzen und Q für $C_1$—$C_6$-Alkoxy steht, dealkyliert wird, wobei eine Verbindung der Formel IX

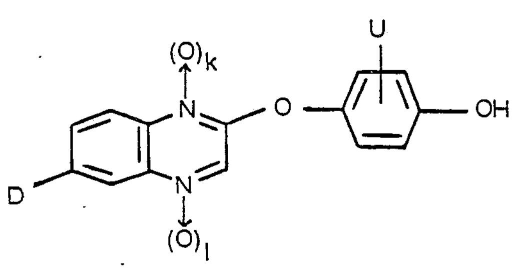

IX

erhalten wird, und dann die Verbindung der formel IX mit einer Verbindung der Formel X

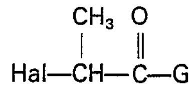

X

worin G die in einem der Ansprüche 1 bis 4 angegebene Bedeutung besitzt und Hal für Chlor, Brom oder Jod steht, umgesetzt wird.

16. Verbindung der Formel VIII

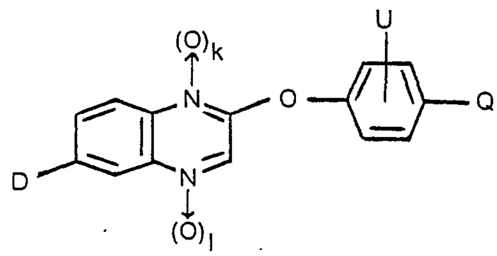

VIII

worin D, U, k und l die in einem der Ansprüche 1 bis 4 angegebenen Bedeutungen besitzen und Q für Hydroxy oder $C_1$—$C_6$-Alkoxy steht.

**Patentansprüche für den Vertragsstaat: AT**

1. Verfahren zur Synthese einer Verbindung der Formel I

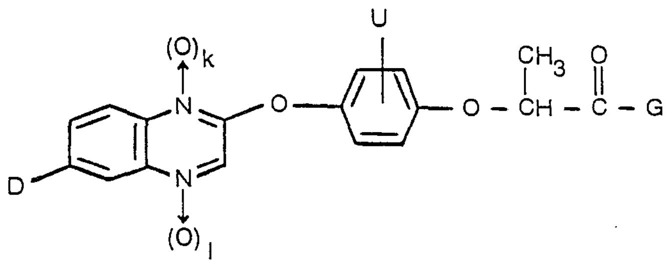

I

oder eines Salz davon, worin

D und U unabhängig ausgewählt sind aus Halogen, Methyl und Halogenomethyl,

G ausgewählt ist aus Hydroxy, Mercapto, $C_1$—$C_{10}$-Alkoxy, $C_2$—$C_{10}$-Alkenyloxy, $C_3$—$C_{10}$-Alkinyloxy, $C_1$—$C_{10}$-Alkylthio, $C_2$—$C_{10}$-Alkenylthio, $C_3$—$C_{10}$-Alkinylthio, $C_3$—$C_7$-Cycloalkoxy under der Gruppe OM, worin M ein Alkalimetall- oder Erdalkalimetallion bedeutet, und

k und l unabhängig ausgewählt sind 0 und 1;

mit der Maßgabe, daß G nicht für Äthoxy steht, wenn D für Chloro steht, k und l für O stehen und U für Fluoro in der 3-Stellung steht,

bei welchem Verfahren entweder

(a) ein Chinoxalinderivat der Formel IX

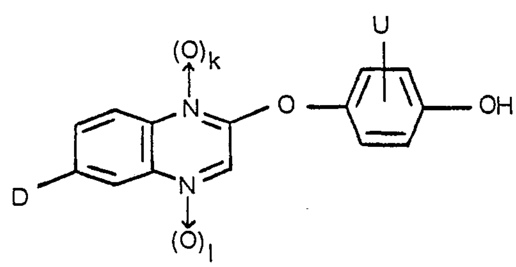

IX

worin D, U, k und l die oben angegebenen Bedeutung besitzen, mit einer Verbindung der Formel X

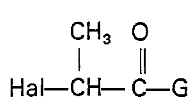

X

worin G die oben angegebene Bedeutung besitzt und Hal für Chlor, Brom oder Jod steht, umgesetzt wird oder

(b) ein Chinoxalinderivat der formel V

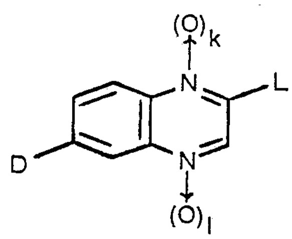

V

worin D, k und l die oben angegebenen Bedeutungen besitzen und L für eine abspaltbare Gruppe steht, mit einer Verbindung der Formel VI

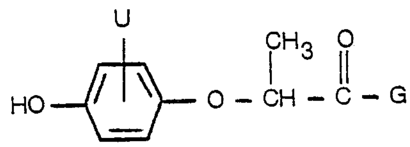

VI

worin U und G die oben angegebenen Bedeutungen besitzen, umgesetzt wird.

2. Verfahren nach Anspruch 1, bei welchem die Chinoxalinverbindung der Formel IX dadurch hergestellt wird, daß ein Chinoxalinderivat der Formel V nach Anspruch 1 mit einem Phenol der Formel VII

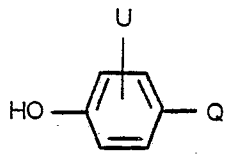

VII

worin U die in Anspruch 1 angegebene Bedeutung besitzt und Q für Hydroxy oder $C_1$—$C_6$-Alkoxy steht, umgesetzt wird, wobei eine Verbindung der Formel VIII

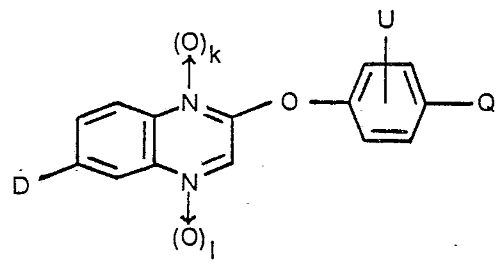

VIII

worin D, U, k und l die in Anspruch 1 angegebenen Bedeutungen besitzen und Q die oben angegebene Bedeutung besitzt, erhalten wird, worauf, wenn Q für $C_1$—$C_6$-Alkoxy steht, die Verbindung der Formel VIII dealkyliert wird, wobei eine Verbindung der Formel IX nach Anspruch 1 erhalten wird.

3. Verfahren nach Anspruch 1 oder 2 zur Synthese einer Verbindung der Formel I oder eines Salzes davon nach Anspruch 1, bei welchem eine Verbindung der Formel VIII

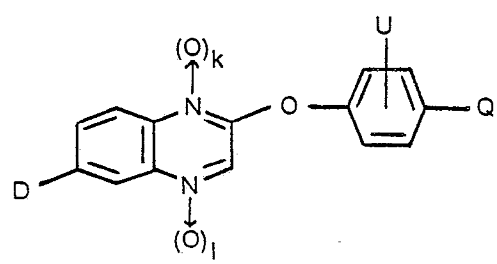

VIII

worin D, U, k und l die in Anspruch 1 angegebenen Bedeutunge besitzen und Q für $C_1$—$C_6$-Alkoxy steht, dealkyliert wird, wobei eine Verbindung der Formel IX

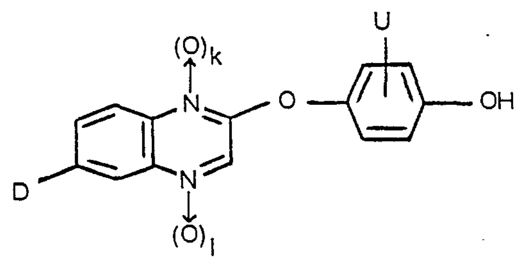

IX

erhalten wird, und dann die Verbindung der Formel IX mit einer Verbindung der Formel X

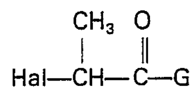

X

worin G die in Anspruch 1 angegebene Bedeutung besitzt und Hal für Chlor, Brom oder Jod steht, umgesetzt wird.

4. Verfahren nach Anspruch 1, 2 oder 3, bei welchem
D ausgewählt ist aus Fluor, Chlor, Brom, Jod und Trifluoromethyl,
U ausgewählt ist aus Fluor, Chlor, Brom und Jod, das in der 2-Stellung des Benzolrings gebunden ist,
G ausgewählt ist aus Hydroxy, $C_1$—$C_6$-Alkoxy, $C_2$—$C_6$-Alkenyloxy, $C_3$—$C_6$-Alkinyloxy, Cyclohexyloxy und der Gruppe OM, worin M ein Alkalimetallion bedeutet, und
k und l beide für O stehen.

5. Verfahren nach einem der vorhergehenden Ansprüche, bei welchem
D ausgewählt ist aus Fluor, Chlor und Brom,
U für Fluor steht, das in der 2-Stellung des Benzolrings gebunden ist,
G ausgewählt ist aus Hydroxy und $C_1$—$C_6$ Alkoxy und
k und l beide für O stehen.

6. Verfahren nach einem der vorhergehenden Ansprüche, bei welchem
D ausgewählt ist aus Fluor und Chlor,
U für Fluor steht, das in der 2-Stellung des Benzolrings gebunden ist,
G ausgewählt ist aus Hydroxy, Äthoxy, n-Propoxy und n-Butoxy und
k und l beide für O stehen.

7. Verfahren nach einem der vorhergehenden Ansprüche, bei welchem die Reaktionsteilnehmer so gewählt werden, daß 2 - [2 - Fluoro - 4 - (6 - chlorochinoxalin - 2 - yloxy)phenoxy]propionsäure - n - propylester erhalten wird.

8. Verfahren nach einem der vorhergehenden Ansprüche, bei welchem die Reaktionsteilnehmer so gewählt werden, daß 2 - [2 - Fluoro - 4 - (6 - fluorochinoxalin - 2 - yloxy)phenoxy]propionsäure - n - propylester erhalten wird.

9. Verfahren nach einem der vorhergehenden Ansprüche, bei welchem eine Verbindung der Formel I oder ein Salz davon in Form eines Enantiomers mit herbicider Aktivität unter weitgehender Abwesenheit des anderen Enantiomers hergestellt wird.

10. Herbicide Zusammensetzung, welche als aktiven Bestandteil eine Verbindung der Formel I nach einem der vorhergehenden Ansprüche und Träger hierfür enthält.

11. Herbicide Zusammensetzung, welche als aktive Bestandteile folgendes enthält:
(a) eine Verbindung der Formel I oder ein Salz davon nach einem der Ansprüche 1 bis 9 und
(b) ein weiteres Herbicid, bei welchem es sich um ein Benzo-2,1,3-thiadiazin-4-on-2,2-dioxid, ein Hormonherbicid, einen 3-[4-(4-Halogenophenoxy)phenyl]-1,1-dialkylharnstoff, ein Dinitrophenol oder ein Derivat davon, ein Dinitroanilinherbicid, ein Phenylharnstoffherbicid, ein Phenylcarbamoyloxyphenylcarbamat, ein 2-Phenylpyridazin-3-on, ein Uracilherbicid, ein Triazinherbicid, ein 1-Alkoxy-1-alkyl-3-phenylharnstoffherbicid, ein Thiolcarbamatherbicid, ein 1,2,4-Triazin-5-on-herbicid, ein benzoesäureherbicid, ein Anilidherbicid, ein Dihalogenobenzonitrilherbicid, ein Halogenoalkansäureherbicid oder ein Salz davon, ein Diphenylätherherbicid, N,N-Dimethyldiphenylacetamid, N(1-Naphthyl)phthalamidsäure, 3-Amino-1,2,4-triazol, ein Bipyridyliumherbicid, ein Organoarsenherbicid oder ein Aminosäureherbicid oder ein Salz oder einen Ester davon handelt.

12. Verfahren zum starken Schädigen oder Abtöten von unerwünschten Pflanzen, bei welchem auf die Pflanzen oder auf das Wachstumsmedium der Pflanzen eine wirksame Menge einer Verbindung, wie sie in einem der Ansprüche 1 bis 9 definiert ist, oder eine wirksame Menge einer Zusammensetzung, wie sie in Ansprüche 10 oder 11 definiert ist, aufgebracht wird.

13. Verfahren zur selektiven Beeinflussung des Wachstums von einkeimblättrigen Unkräutern in

zweikeimblättrigen Anpflanzungen, bei welchem auf die Anpflanzung oder auf das Wachstumsmedium der Anpflanzung eine Verbindung, wie sie in einem der Ansprüche 1 bis 9 definiert ist, oder eine Zusammensetzung, wie sie in Anspruch 10 oder 11 definiert ist, in einer Menge aufgebracht wird, die ausreicht, die Unkräuter stark zu schädigen oder abzutöten, die aber nicht ausreicht, die Anpflanzung wesentlich zu schädigen.

14. Verfahren nach Anspruch 12 oder 13, bei welchem die Verbindung in einer Rate im Bereich von 0,005 bis 20 kg/ha aufgebracht wird.

**Patentansprüche für den Vertragsstaat: LU**

1. Verbindung der Formel I

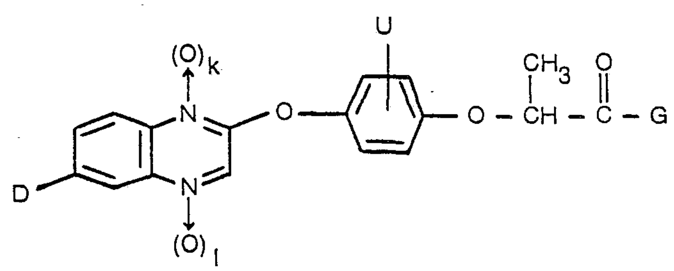

I

oder ein Salz davon, worin

D und U unabhängig ausgewählt sind aus Halogen, Methyl und Halogenomethyl,

G ausgewählt ist aus Hydroxy, Mercapto, $C_1$—$C_{10}$-Alkoxy, $C_2$—$C_{10}$-Alkenyloxy, $C_3$—$C_{10}$-Alkinyloxy, $C_1$—$C_{10}$-Alkylthio, $C_2$—$C_{10}$-Alkenylthio, $C_3$—$C_{10}$-Alkinylthio, $C_3$—$C_7$-Cycloalkoxy under der Gruppe OM, worin M ein Alkalimetall- oder Erdalkalimetallion bedeutet, und

k und l unabhängig ausgewählt sind 0 und 1.

2. Verbindung nach Anspruch 1, worin

D ausgewählt ist aus Fluor, Chlor, Brom, Jod und Trifluoromethyl,

U ausgewählt ist aus Fluor, Chlor, Brom und Jod, das in der 2-Stellung des Benzolrings gebunden ist,

G ausgewählt ist aus Hydroxy, $C_1$—$C_6$-Alkoxy, $C_2$—$C_6$-Alkenyloxy, $C_3$—$C_6$-Alkinyloxy, Cyclohexyloxy und der Gruppe OM, worin M ein alkalimetallion bedeutet, und

k und l beide für O stehen.

3. Verbindung nach Anspruch 1 oder 2, worin

D ausgewählt ist aus Fluor, Chlor und Brom,

U für Fluor steht, das in der 2-Stellung des Benzolrings gebunden ist,

G ausgewählt ist aus Hydroxy und $C_1$—$C_6$ Alkoxy und

k und l beide für O stehen.

4. Verbindung nach einem der Ansprüche 1 bis 3, worin D ausgewählt ist aus Fluor und Chlor,

U für Fluor steht, das in der 2-Stellung des Benzolrings gebunden ist,

G ausgewählt ist aus Hydroxy, Äthoxy, n-Propoxy und n-Butoxy und

k und l beide für O stehen.

5. Die Verbindung 2 - [2 - Fluoro - 4 - (6 - chlorochinoxalin - 2 - yloxy)phenoxy]propionsäure - n - propylester.

6. Die Verbindung 2 - [2 - Fluoro - 4 - (6 - fluorochinoxalin - 2 - yloxy)phenoxy]propionsäure - n - propylester.

7. Zusammensetzung mit herbicider Aktivität gegen einkeimblättrige Unkräuter in zweikeimblättrigen Anpflanzungen, dadurch gekennzeichnet, daß sie im wesentlichen aus einem Enantiomer einer Verbindung der Formel I oder einem Salz davon nach einem der Ansprüche 1 bis 6 besteht, wobei die Zusammensetzung im wesentlichen frei vom anderen Enantiomer ist.

8. Herbicide Zusammensetzung, welche als aktiven Bestandteil eine Verbindung nach einem der Ansprüche 1 bis 6 und einen Träger hierfür enthält.

9. Herbicide Zusammensetzung, welche als aktive Bestandteile folgendes enthält:

(a) eine Verbindung der Formel I oder ein Salz davon nach einem der Ansprüche 1 bis 6 und

(b) ein weiteres Herbicid, bei welchem es sich um ein Benzo-2,1,3-thiadiazin-4-on-2,2-dioxid, ein Hormonherbicid, einen 3-[4-(4-Halogenophenoxy)phenyl]-1,1-dialkylharnstoff, ein Dinitrophenol oder ein Derivat davon, ein Dinitroanilinherbicid, ein Phenylharnstoffherbicid, ein Phenylcarbamoyloxyphenylcarbamat, ein 2-Phenylpyridazin-3-on, ein Uracilherbicid, ein Triazinherbicid, ein 1-Alkoxy-1-alkyl-3-phenylharnstoffherbicid, ein Thiolcarbamatherbicid, ein 1,2,4-Triazin-5-on-herbicid, ein Benzoesäureherbicid, ein Anilidherbicid, ein Dihalogenobenzonitrilherbicid, ein Halogenoalkansäureherbicid oder ein Salz davon, ein Diphenylätherherbicid, N,N-Dimethyldiphenylacetamid, N(1-Naphthyl)phthalamidsäure, 3-Amino-1,2,4-triazol, ein Bipyridyliumherbicid, ein Organoarsenherbicid oder ein Aminosäureherbicid oder ein Salz oder einen Ester davon handelt.

10. Verfahren zum starken Schädigen oder Abtöten von unerwünschten Pflanzen, bei welchem auf die Pflanzen oder auf das Wachstumsmedium der Pflanzen eine wirksame Menge einer Verbindung nach einem der Ansprüche 1 bis 6 oder eine wirksame Menge einer Zusammensetzung nach einem der Ansprüche 7 bis 9 aufgebracht wird.

11. Verfahren zur selektiven Bekämpfung des Wachstums von einkeimblättrigen Unkräutern in zweikeimblättrigen Anpflanzungen, bei welchem auf die Anpflanzung oder auf das Wachstumsmedium der Anpflanzung eine Verbindung nach einem der Ansprüche 1 bis 6 oder eine Zusammensetzung nach einem der Ansprüche 7 bis 9 in einer Menge aufgebracht wird, die ausreicht, die Unkräuter stark zu schädigen oder abzutöten, die aber nicht ausreicht, die Anpflanzung wesentlich zu schädigen.

12. Verfahren nach Anspruch 10 oder 11, bei welchem die Verbindung in einer Rate im Bereich von 0,005 bis 20 kg/ha aufgebracht wird.

13. Verfahren zur Synthese einer Verbindung der Formel I nach einem der Ansprüche 1 bis 6, bei welchem entweder ein Chinoxalinderivat der formel IX mit einer Verbindung der Formel X

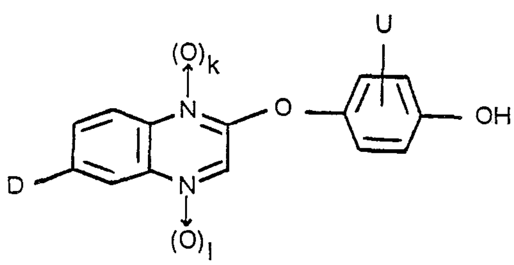

IX

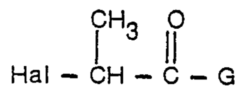

X

worin D, U, G, k und l die in einer der Ansprüche 1 bis 4 angegebenen Bedeutungen bewitzen und Hal für Chlor, Brom oder Jod steht, umgesetzt wird oder ein Chinoxalinderivat der Formel V einer Verbindung der Formel VI

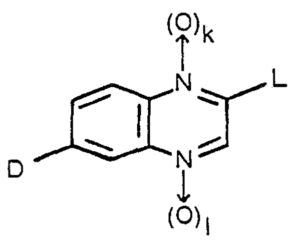

\+

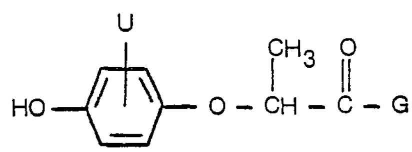

V VI

worin D, U, G, k und l die einem der Ansprüche 1 bis 4 angegebenen Bedeutungen besitzen und L für eine abspaltbare Gruppe steht, umgesetzt wird.

14. Verfahren nach Anspruch 13, bei welchem die Chinoxalinverbindung der Formel IX durch Umsetzung eines Chinoxalinderivats der Formel V nach Anspruch 13 mit einem Phenol der Formel VII

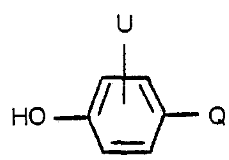

VII

worin U die in einem der Ansprüche 1 bis 4 angegebene Bedeutung besitzt und Q für Hydroxy oder $C_1$—$C_6$-Alkoxy steht, umgesetzt wird, wobei eine Verbindung der Formel VIII

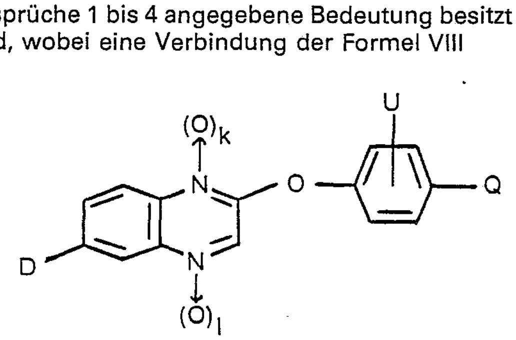

VIII

worin D, U, k und l die in einem der Ansprüche 1 bis 4 angegebenen Bedeutungen besitzen und Q die oben angegebene Bedeutung besitzt, erhalten wird, worauf, wenn Q für $C_1$—$C_6$-Alkoxy steht, die Verbindung der Formel VIII dealkyliert wird, wobei eine Verbindung der Formel IX nach Anspruch 13 erhalten wird.

15. Verfahren zur Synthese einer Verbindung der Formel I oder eines Salzes davon nach einem der Ansprüche 1 bis 6, bei welchem eine Verbindung der Formel VIII

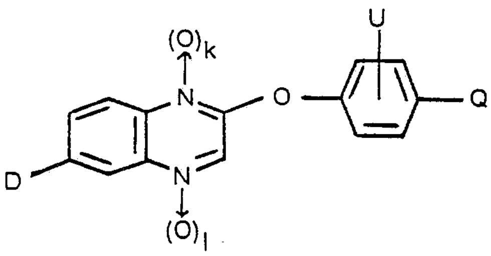

VIII

worin D, U, k und l die in einem der Ansprüche 1 bis 4 angegebenen Bedeutungen besitzen und Q für $C_1$—$C_6$-Alkoxy steht, dealkyliert wird, wobei eine Verbindung der Formel IX

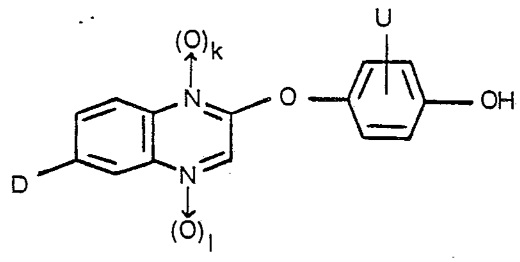

IX

erhalten wird, und dann die Verbindung der Formel IX mitr einer Verbindung der Formel X

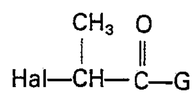

X

worin G die in einem der Ansprüche 1 bis 4 angegebene Bedeutung besitzt und Hal für Chlor, Brom oder Jod steht, umgesetzt wird.

16. Verbindung der Formel VIII

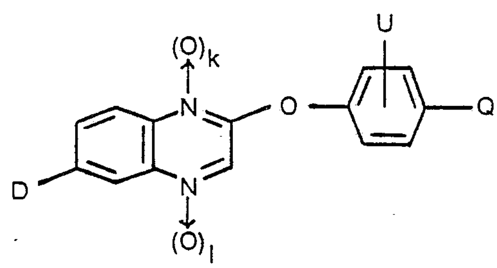

VIII

worin D, U, k und l die in einem der Ansprüche 1 bis 4 angegebenen Bedeutungen besitzenund Q für Hydroxy oder $C_1$—$C_6$-Alkoxy steht.

**Revendications pour les Etats contractants: FR BE CH DE GB IT LI NL SE**

1. Composé de formule I:

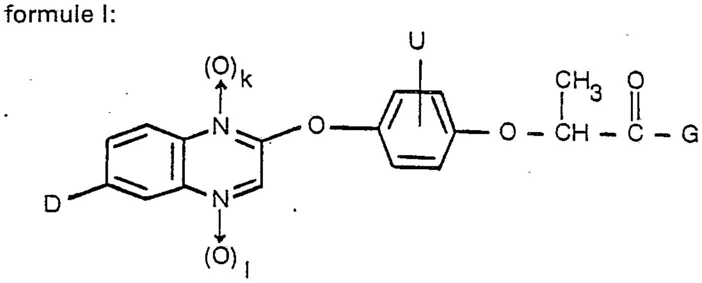

I

ou un sel de ce composé, formule dans laquelle:

D et U sont choisis indépendamment entre un halogène, un groupe méthyle et un groupe halogénométhyle;

G est choisi entre un groupe hydroxy, mercapto, alkoxy en $C_1$ à $C_{10}$, alcényloxy en $C_2$ à $C_{10}$, alcynyloxy en $C_3$ à $C_{10}$, alkylthio en $C_1$ à $C_{10}$, alcénylthio en $C_2$ à $C_{10}$, alcynylthio en $C_3$ à $C_{10}$, cycloalkoxy en $C_3$ à $C_7$, et le groupe OM dans lequel M est un ion de métal alcalin ou un ion de métal alcalino-terreux; et

k et l sont choisis indépendamment entre 0 et 1; sous réserve que lorsque D est le radical chloro, k et l sont égaux à O et U est un radical fluoro en position 3, G ne soit pas un groupe éthoxy.

2. Composé suivant la revendication 1, dans lequel:

D est choisi entre le fluor, le chlore, le brome, l'iode et le groupe trifluorométhyle;

U est choisi entre le fluor, le chlore, le brome et l'iode substitués en position 2 du noyau benzénique;

G est choisi entre un groupe hydroxy, alkoxy en $C_1$ à $C_6$, alcényloxy en $C_2$ à $C_6$, alcynyloxy en $C_3$ à $C_6$, cyclohexyloxy et le groupe OM dans lequel M est un ion de métal alcalin; et

k et l sont tous deux égaux à 0.

3. Composé suivant la revendication 1 ou 2, dans lequel:

D est choisi entre le fluor, le chlore et le brome;

U est le fluor substitué en position 2 du noyau benzénique;

G est choisi entre un groupe hydroxy et alkoxy en $C_1$ à $C_6$; et

k et l sont deux égaux à 0.

4. Composé suivant l'une quelconque des revendications 1 à 3, dans lequel:

D est choisi entre le fluor et le chlore;

U est le fluor substitué en position 2 du noyau benzénique;

G est choisi entre les groupes hydroxy, éthoxy, n-propoxy et n-butoxy; et

k et l sont tous deux égaux à 0.

5. Le 2-[2-fluoro-4-(6-chloroquinoxaline-2-yloxy)phénoxy]propionate de n-propyle.

6. Le 2-[2-fluoro-4-(6-fluoroquinoxaline-2-yloxy)phénoxy]propionate de n-propyle.

7. Composition douée d'activité herbicide contre des mauvaises herbes monocotylédones dans des plantes cultivées dicotylédones, caractérisée en ce qu'elle consiste essentiellement en un énantiomère d'un composé de formule I ou un sel de ce composé suivant l'une quelconque des revendications 1 à 6, ladite composition étant pratiquement dépourvue de l'autre énantiomère.

8. Composition herbicide comprenant comme ingrédient actif un composé suivant l'une quelconque des revendications 1 à 6 et un support approprié.

9. Composition herbicide, comprenant comme ingrédients actifs,

(a) un composé de formule I ou un sel de ce composé suivant l'une quelconque des revendications 1 à 6; et

(b) un autre herbicide qui est un benzo-2,1,3-thiadiazine-4-one-2,2-dioxyde; un herbicide hormonal; une 3-[4-(4-halogénophénoxy)phényl]-1,1-dialkylurée; un dinitrophénol ou un dérivé de ce composé; un herbicide du type dinitroaniline; un herbicide du type phénylurée; un phénylcarbamoyloxyphénylcarbamate; une 2-phénylpyridazine-3-one; un herbicide du type uracile; un herbicide du type triazine; un herbicide du type 1-alkoxy-1-alkyl-3-phénylurée; un herbicide du type thiolcarbamate un herbicide du type 1,2,4-triazine-5-one; un herbicide du type acide benzoïque; un herbicide du type anilide; un herbicide du type dihalogénobenzonitrile; un herbicide du type acide halogénoalcanoïque ou un sel de cet acide; un herbicide du type d'éther de diphényle; le N,N-diméthyldiphénylacétamide; l'acide N(1-naphtyl)-phtalamique; le 3-amino-1,2,4-triazole; un herbicide du type bipyridylium; un herbicide organoarsénié; ou un herbicide du type d'un aminoacide ou un sel ou ester de ce composé.

10. Procédé pour endommager gravement ou détruire des plantes indésirables, procédé qui consiste à appliquer auxdites plantes, ou à leur milieu de croissance, une quantité efficace d'un composé suivant l'une quelconque des revendications 1 à 6 ou une quantité efficace d'une composition suivant l'une quelconque des revendications 7 à 9.

11. Procédé pour inhiber sélectivement la croissance de mauvaises herbes monocotylédones dans des plantes cultivées dicotylédones, procédé qui consiste à appliquer à ladite plante cultivée, ou à son milieu de croissance, un composé suivant l'une quelconque des revendications 1 à 6 ou une composition suivant l'une quelconque des revendications 7 à 9 en une quantité suffisante pour altérer gravement ou détruire les mauvaises herbes mais insuffisante pour altérer gravement la plante cultivée.

12. Procédé suivant la revendication 10 ou la revendication 11, dans lequel le composé est appliqué à un taux compris dans l'intervalle de 0,005 à 20 kg/hectare.

13. Procédé de synthèse d'un composé de formule I suivant l'une quelconque des revendications 1 à 6, procédé qui consiste à faire réagir un dérivé de quinoxaline de formule IX avec un composé de formule X:

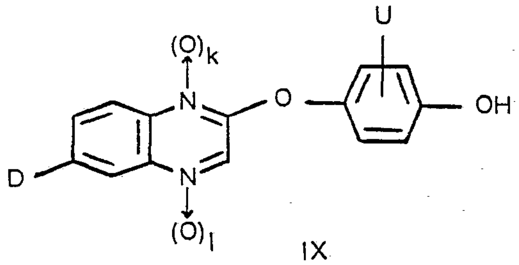

IX

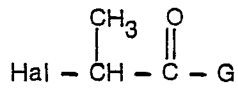

X

dans laquelle D, U, G, k et l ont les définitions données dans l'une quelconque des revendications 1 à 4, et hal est le chlore, le brome ou l'iode; ou la réaction d'un dérivé de quinoxaline de formule V avec un composé de formule VI:

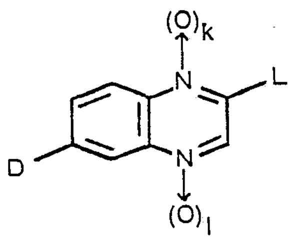

+

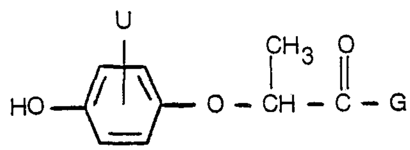

V VI

dans lesquelles D, U, G, k et l ont les définitions données dans l'une quelconque des revendications 1 à 4 et L est un groupe partant.

14. Procédé suivant la revendication 13, dans lequel le composé de quinoxaline de formule IX est préparé par réaction d'un dérivé de quinoxaline de formule V comme défini dans la revendication 13, avec un phénol de formule VII:

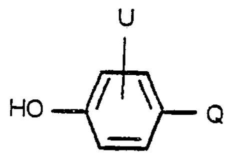

VII

dans laquelle U a la définition donnée dans l'une quelconque des revendications 1 à 4 et Q est un groupe hydroxy ou un groupe alkoxy en $C_1$ à $C_6$, pour obtenir un composé de formule VIII

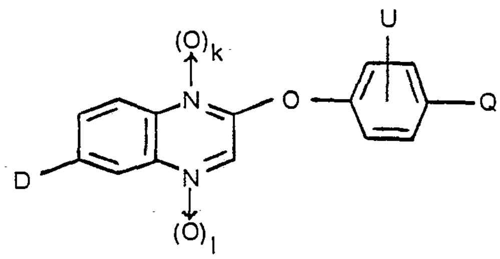

VIII

dans laquelle D, U, k et l ont les définitions données dans l'une quelconque des revendications 1 à 4 et Q a la définition donnée ci-dessus, et lorsque Q est un groupe alkoxy en $C_1$ à $C_6$, à désalkyler le composé de formule VIII pour obtenir un composé de formule IX comme défini dans la revendication 13.

15. Procédé de synthèse d'un composé de formule I ou d'un sel de ce composé suivant l'une quelconque des revendications 1 à 6, qui consiste à désalkyler un composé de formule VIII:

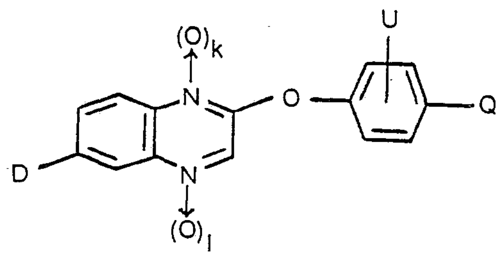

VIII

dans laquelle D, U, k et l ont les définitions données dans l'une quelconque des revendications 1 à 4 et Q est un groupe alkoxy en $C_1$ à $C_6$ pour former un composé de formule IX:

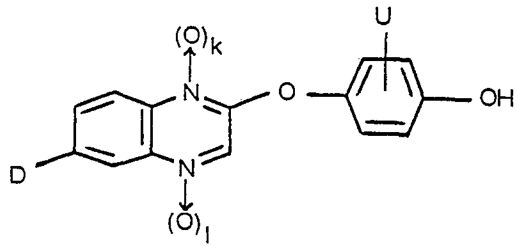

IX

puis à faire réagir le composé de formule IX avec un composé de formule X:

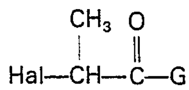

X

dans laquelle G est tel que défini dans l'une quelconque des revendications 1 à 4 et hal est le chlore, le brome ou l'iode.

16. Composé de formule VIII:

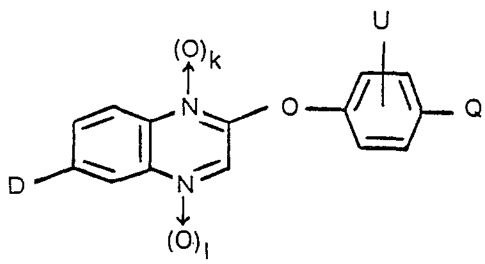

VIII

dans laquelle D, U, k et l sont tels que définis dans l'une quelconque des revendications 1 à 4 et Q est un groupe hydroxy ou un groupe alkoxy en $C_1$ à $C_6$.

**Revendications pour l'Etat contractant: AT**

1. Procédé de synthèse d'un composé de formule

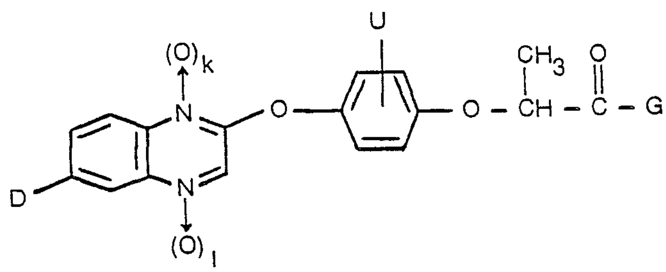

I

ou d'un sel de ce composé: formule dans laquelle:

D et U sont choisis indépendamment entre un halogène, un groupe méthyle et un groupe halogénométhyle;

G est choisi entre un groupe hydroxy, mercapto, alkoxy en $C_1$ à $C_{10}$, alcényloxy en $C_2$ à $C_{10}$, alcynyloxy en $C_3$ à $C_{10}$, alkylthio en $C_1$ à $C_{10}$, alcénylthio en $C_2$ à $C_{10}$, alcynylthio en $C_3$ à $C_{10}$, cycloalkoxy en $C_3$ à $C_7$, et le groupe OM dans lequel M est un ion de métal alcalin ou un ion de métal alcalino-terreux; et

k et l sont choisis indépendamment entre 0 et 1; sous réserve que lorsque D est le radical chloro, k et l sont égaux à O et U est un radical fluoro en position 3, G ne soit pas un groupe éthoxy, procédé qui consiste:

(a) à faire réagir un dérivé de quinoxaline de formule IX:

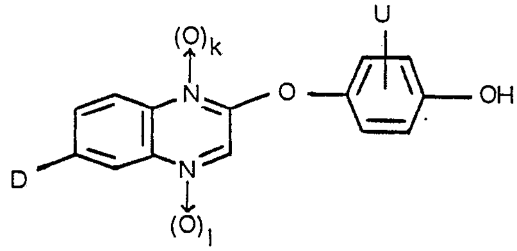

IX

dans laquelle D, U, k et l ont les définitions données ci-dessus, avec un composé de formule X:

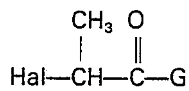

X

dans laquelle G est tel que défini ci-dessus et hal représente le chlore, le brome ou l'iode; ou

(b) à faire réagir un dérivé de quinoxaline de formule V:

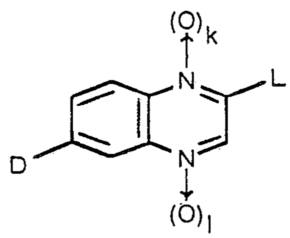

V

dans laquelle D, k et l ont les définitions données ci-dessus et L est un groupe partant, avec un composé de formule VI:

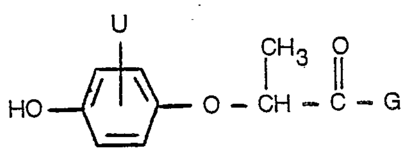

VI

dans laquelle U et G sont tels que définis ci-dessus.

2. Procédé suivant la revendication 1, dans lequel le composé de quinoxaline de formule IX est préparé par réaction d'un dérivé de quinoxaline de formule V tel que défini dans la revendication 1, avec un phénol de formule VII:

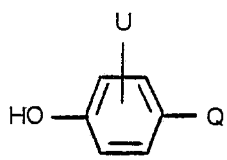

VII

dans laquelle U a la définition donnée dans la revendication 1 et Q est un groupe hydroxy ou alkoxy en $C_1$ à $C_6$, pour former un composé de formule VIII:

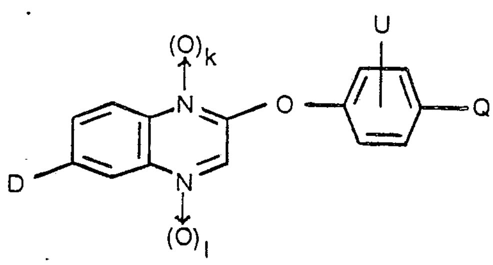

VIII

dans laquelle D, U, k et l ont les définitions données dans la revendication 1 et Q a la définition donnée ci-dessus et, lorsque Q est un groupe alkoxy en $C_1$ à $C_6$, à désalkyler le composé de formule VIII pour obtenir un composé de formule IX tel que défini dans la revendication 1.

3. Procédé suivant la revendication 1 ou 2, pour la synthèse d'un composé de formule I ou d'un sel de ce composé comme défini dans la revendication 1, qui consiste à désalkyler un composé de formule VIII:

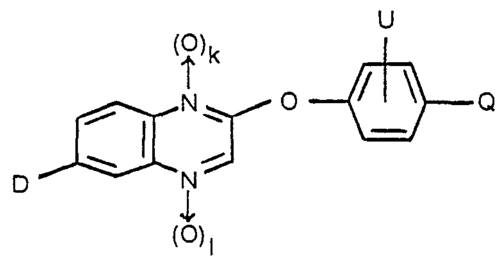

VIII

dans laquelle D, U, k et l ont la définition donnée dans la revendication 1 et Q est un groupe alkoxy en $C_1$ à $C_6$, pour obrenir un composé de formule IX:

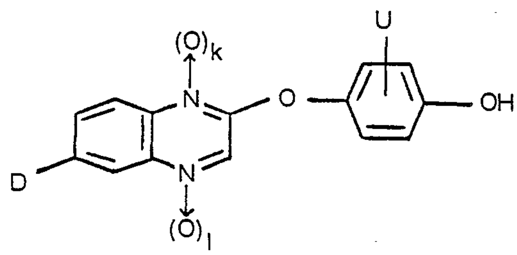

IX

puis à faire réagir le composé de formule IX avec un composé de formule X:

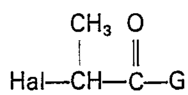

X

dans laquelle G est tel que défini dans la revendication 1 et hal est le chlore, le brome ou l'iode.

4. Procédé suivant les revendications 1, 2 ou 3, dans lequel:

D est choisi entre le fluor, le chlore, le brome, l'iode et le groupe trifluorométhyle;

U est choisi entre le fluor, le chlore, le brome et l'iode substitués en position 2 du noyau benzénique;

G est choisi entre des groupes hydroxy, alkoxy en $C_1$ à $C_6$, alcényloxy en $C_2$ à $C_6$, alcynyloxy en $C_3$ à $C_6$, cyclohexyloxy et le groupe OM dans lequel M est un ion de métal alcalin; et

k et l sont tous deux égaux à 0.

5. Procédé suivant l'une quelconque des revendications précédentes, dans lequel:

D est choisi entre le fluor, le chlore et le brome;

U est le fluor substitué en position 2 du noyau benzénique;

G est choisi entre un groupe hydroxy et un groupe alkoxy en $C_1$ à $C_6$; et

k et l sont tous deux égaux à 0.

6. Procédé suivant l'une quelconque des revendications précédentes, dans lequel:

D est choisi entre le fluor et le chlore;

U est le fluor substitué en position 2 du noyau benzénique.

G est choisi entre les groupes hydroxy, éthoxy, n-propoxy et n-butoxy; et

k et l sont tous deux égaux à 0.

7. Procédé suivant l'une quelconque des revendications précédentes, dans lequel les corps réactionnels sont choisis de manière à produire le 2-[2-fluoro-4-(6-chloroquinoxaline-2-yloxy)phénoxy]-propionate de n-propyle.

8. Procédé suivant l'une quelconque des revendications précédentes, dans lequel les corps réactionnels sont choisis de manière à produire le 2-[2-fluoro-4-(6-fluoroquinoxaline-2-yloxy)phénoxy]-propionate de n-propyle.

9. Procédé suivant l'une quelconque des revendications précédentes, dans lequel un composé de formule I ou un sel de ce composé est produit sous la forme d'un énantiomère doué d'activité herbicide, en l'absence quasi-totale de l'autre énantiomère.

10. Composition herbicide comprenant comme ingrédient actif un composé de formule I tel que défini dans l'une quelconque des revendications précédentes et un support approprié.

11. Composition herbicide comprenant comme ingrédients actifs:

(a) un composé de formule I ou un sel tel que défini dans l'une quelconque des revendications 1 à 9; et

(b) un autre herbicide qui est un benzo-2,1,3-thiadiazine-4-one-2,2-dioxyde; un herbicide hormonal; une 3-[4-(4-halogénophénoxy)phényl]-1,1-dialkylurée; un dinitrophénol ou un dérivé de ce composé; un herbicide du type dinitroaniline; un herbicide du type phénylurée; un phénylcarbamoyloxyphénylcarbamate; une 2-phénylpyridazine-3-one; un herbicide du type uracile; un herbicide du type triazine; un herbicide du type 1-alkoxy-1-alkyl-3-phénylurée; un herbicide du type thiolcarbamate un herbicide du type 1,2,4-triazine-5-one; un herbicide du type acide benzoïque; un herbicide du type anilide; un herbicide du type dihalogénobenzonitrile; un herbicide du type acide halogénoalcanoïque ou un sel de cet acide; un herbicide du type d'éther de diphényle; le N,N-diméthyldiphénylacétamide; l'acide N(1-naphtyl)-phtalamique; le 3-amino-1,2,4-triazole; un herbicide du type bipyridylium; un herbicide organoarsénié; ou un herbicide du type d'un aminoacide ou un sel ou ester de ce composé.

12. Procédé pour endommager gravement ou détruire des plantes indésirables, procédé qui consiste à appliquer auxdites plantes, ou à leur milieu de croissance, une quantité efficace d'un composé tel que défini dans l'une quelconque des revendications 1 à 9 ou une quantité efficace d'une composition suivant la revendication 10 ou 11.

13. Procédé pour combattre sélectivement la croissance de mauvaises herbes monocotylédones dans des plantes cultivées dicotylédones, procédé qui consiste à appliquer à ladite plante cultivée, ou à son milieu de croissance, un composé tel que défini dans l'une quelconque des revendications 1 à 9 ou une composition suivant la revendication 10 ou 11 en une quantité suffisante pour endommager gravement ou détruire les mauvaises herbes mais insuffisante pour endommager notablement la plante cultivée.

14. Procédé suivant la revendication 12 ou la revendication 13, dans lequel le composé est appliqué à un taux compris dans l'intervalle de 0,005 à 20 kg/hectare.

**Revendications pour l'Etat contractant: LU**

1. Composé de formule I:

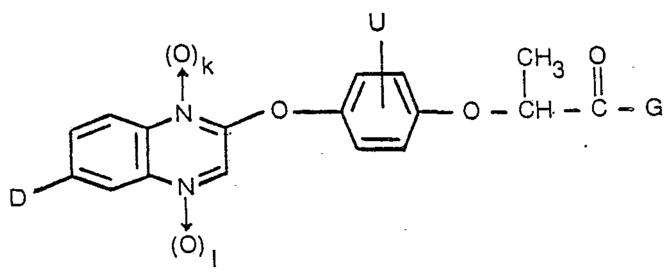

I

ou un sel de ce composé, formule dans laquelle:

D et U sont choisis indépendamment entre un halogène, un groupe méthyle et un groupe halogénométhyle;

G est choisi entre un groupe hydroxy, mercapto, alkoxy en $C_1$ à $C_{10}$, alcényloxy en $C_2$ à $C_{10}$, alcynyloxy en $C_3$ à $C_{10}$, alkylthio en $C_1$ à $C_{10}$, alcénylthio en $C_2$ à $C_{10}$, alcynylthio en $C_3$ à $C_{10}$, cycloalkoxy en $C_3$ à $C_7$, et le groupe OM dans lequel M est un ion de métal alcalin ou un ion de métal alcalino-terreux; et

k et l sont choisis indépendamment entre 0 et 1.

2. Composé suivant la revendication 1, dans lequel:

D est choisi entre le fluor, le chlore, le brome, l'iode et le groupe trifluorométhyle;

U est choisi entre le fluor, le chlore, le brome et l'iode substitués en position 2 du noyau benzénique;

G est choisi entre un groupe hydroxy, alkoxy en $C_1$ à $C_6$, alcényloxy en $C_2$ à $C_6$, alcynyloxy en $C_3$ à $C_6$, cyclohexyloxy et le groupe OM dans lequel M est un ion de métal alcalin; et

k et l sont tous deux égaux à 0.

3. Composé suivant la revendication 1 ou 2, dans lequel:

D est choisi entre le fluor, le chlore et le brome;

U est le fluor substitué en position 2 du noyau benzénique;

G est choisi entre un groupe hydroxy et alkoxy en $C_1$ à $C_6$; et

k et l sont deux égaux à 0.

4. Composé suivant l'une quelconque des revendications 1 à 3, dans lequel:

D est choisi entre le fluor et le chlore;

U est le fluor substitué en position 2 du noyau benzénique;

G est choisi entre les groupes hydroxy, éthoxy, n-propoxy et n-butoxy; et

k et l sont tous deux égaux à 0.

5. Le 2-[2-fluoro-4-(6-chloroquinoxaline-2-yloxy)phénoxy]propionate de n-propyle.

6. Le 2-[2-fluoro-4-(6-fluoroquinoxaline-2-yloxy)phénoxy]propionate de n-propyle.

7. Composition douée d'activité herbicide contre des mauvaises herbes monocotylédones dans des plantes cultivées dicotylédones, caractérisée en ce qu'elle consiste essentiellement en un énantiomère

d'un composé de formule I ou un sel de ce composé suivant l'une quelconque des revendications 1 à 6, ladite composition étant pratiquement dépourvue de l'autre énantiomère.

8. Composition herbicide comprenant comme ingrédient actif un composé suivant l'une quelconque des revendications 1 à 6 et un support approprié.

9. Composition herbicide, comprenant comme ingrédients actifs,

(a) un composé de formule I ou un sel de ce composé suivant l'une quelconque des revendications 1 à 6; et

(b) un autre herbicide qui est un benzo-2,1,3-thiadiazine-4-one-2,2-dioxyde; un herbicide hormonal; une 3-[4-(4-halogénophénoxy)phényl]-1,1-dialkylurée; un dinitrophénol ou un dérivé de ce composé; un herbicide du type dinitroaniline; un herbicide du type phénylurée; un phénylcarbamoyloxyphénylcarbamate; une 2-phénylpyridazine-3-one; un herbicide du type uracile; un herbicide du type triazine; un herbicide du type 1-alkoxy-1-alkyl-3-phénylurée; un herbicide du type thiolcarbamate un herbicide du type 1,2,4-triazine-5-one; un herbicide du type acide benzoïque; un herbicide du type anilide; un herbicide du type dihalogénobenzonitrile; un herbicide du type acide halogénoalcanoïque ou un sel de cet acide; un herbicide du type d'éther de diphényle; le N,N-diméthyldiphénylacétamide; l'acide N(1-naphtyl)-phtalamique; le 3-amino-1,2,4-triazole; un herbicide du type bipyridylium; un herbicide organoarsénié; ou un herbicide du type d'un aminoacide ou un sel ou ester de ce composé.

10. Procédé pour endommager gravement ou détruire des plantes indésirables, procédé qui consiste à appliquer auxdites plantes, ou à leur milieu de croissance, une quantité efficace d'un composé suivante l'une quelconque des revendications 1 à 6 ou une quantité efficace d'une composition suivant l'une quelconque des revendications 7 à 9.

11. Procédé pour inhiber sélectivement la croissance de mauvaises herbes monocotylédones dans des plantes cultivées dicotylédones, procédé qui consiste à appliquer à ladite plante cultivée, ou à son milieu de croissance, un composé suivant l'une quelconque des revendications 1 à 6 ou une composition suivant l'une quelconque des revendications 7 à 9 en une quantité suffisante pour altérer gravement ou détruire les mauvaises herbes mais insuffisante pour altérer gravement la plante cultivée.

12. Procédé suivant la revendication 10 ou la revendication 11, dans lequel le composé est appliqué à un taux compris dans l'intervalle de 0,005 à 20 kg/hectare.

13. Procédé de synthèse d'un composé de formule I suivant l'une quelconque des revendications 1 à 6, procédé qui consiste à faire réagir un dérivé de quinoxaline de formule IX avec un composé de formule X:

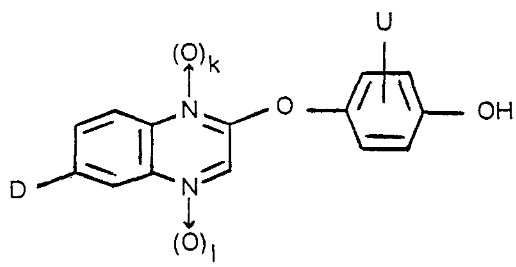

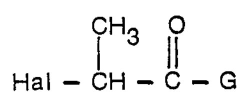

IX X

dans laquelle D, U, G, k et l ont les définitions données dans l'une qelconque des revendications 1 à 4, et hal est le chlore, le brome ou l'iode; ou la réaction d'un dérivé de quinoxaline de formule V avec un composé de formule VI:

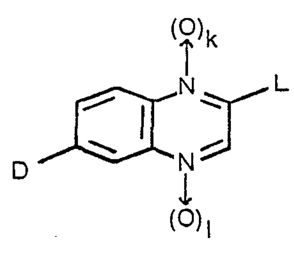

+

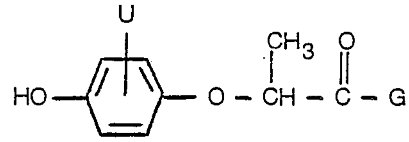

V VI

dans lesquelles D, U, G, k et l ont les définitions données dans l'une quelconque des revendications 1 à 4 et L est un groupe partant.

14. Procédé suivant la revendication 13, dans lequel le composé de quinoxaline de formule IX est préparé par réaction d'un dérivé de quinoxaline de formule V comme défini dans la revendication 13, avec un phénol de formule VII:

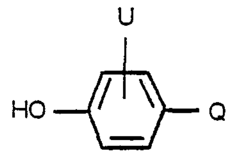

VII

dans laquelle U a la définition donnée dans l'une quelconque des revendications 1 à 4 et Q est un groupe hydroxy ou un groupe alkoxy en $C_1$ à $C_6$, pour obtenir un composé de formule VIII

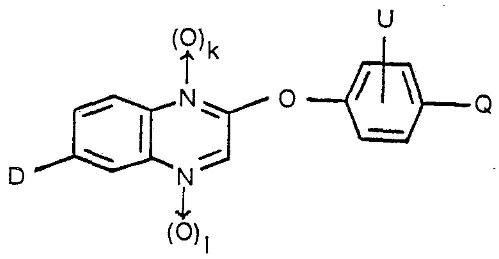

VIII

dans laquelle D, U, k et l ont les définitions données dans l'une quelconque des revendications 1 à 4 et Q a la définition donnée ci-dessus, et lorsque Q est un groupe alkoxy en $C_1$ à $C_6$, à désalkyler le composé de formule VIII pour obtenir un composé de formule IX comme défini dans la revendication 13.

15. Procédé de synthèse d'un composé de formule I ou d'un sel de ce composé suivant l'une quelconque des revendications 1 à 6, qui consiste à désalkyler un composé de formule VIII:

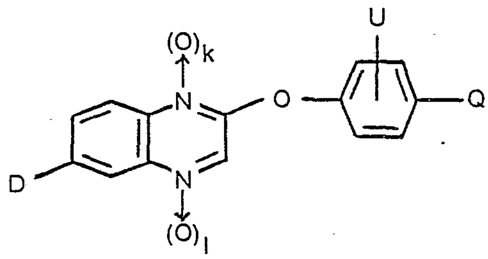

VIII

dans laquelle D, U, k et l ont les définitions données dans l'une quelconque des revendications 1 à 4 et Q est un groupe alkoxy en $C_1$ à $C_6$ pour former un composé de formule IX:

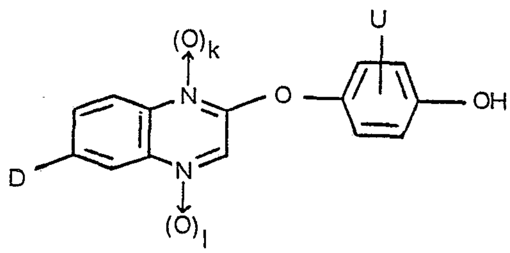

IX

puis à faire réagir le composé de formule IX avec un composé de formule X:

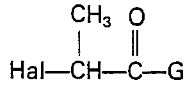

X

dans laquelle G est tel que défini dans l'une quelconque des revendications 1 à 4 et hal est le chlore, le brome ou l'iode.

16. Composé de formule VIII:

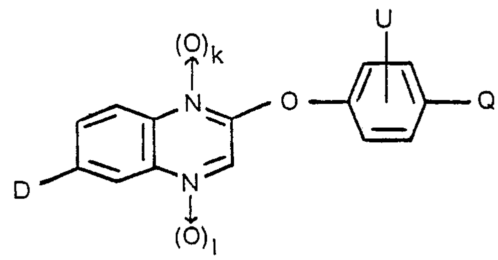

VIII

dans laquelle D, U, k et l sont tels que définis dans l'une quelconque des revendications 1 à 4 et Q est un groupe hydroxy ou un groupe alkoxy en $C_1$ à $C_6$.